# EUROPEAN PATENT SPECIFICATION

(11) **EP 3 271 377 B1**
(45) Date of publication and mention of the grant of the patent: **13.03.2019**
(21) Application number: 16709392.1
(22) Date of filing: 10.03.2016
(51) Int. Cl.: A61K 38/00, C07K 14/47, G01N 33/68

(54) **A PEPTIDE OR COLLECTION OF PEPTIDES DERIVED FROM AMYLOID PRECURSOR PROTEIN**
PEPTID ODER SAMMLUNG VON PEPTIDEN AUS AMYLOID-VORLÄUFERPROTEIN
PEPTIDE OU COLLECTION DE PEPTIDES DÉRIVÉS DE PROTÉINE PRÉCURSEUR DE L'AMYLOÏDE

(30) Priority: 19.03.2015 EP 15159877
(43) Date of publication of application: 24.01.2018
(73) Proprietor: Ludwig-Maximilians-Universität München, 80539 München (DE); Deutsches Zentrum für Neurodegenerative Erkrankungen e.V. (DZNE), 53127 Bonn (DE)
(72) Inventor: WILLEM, Michael, 81245 München (DE); HAASS, Christian, 82057 Icking (DE)
(74) Representative: Engelhard, Markus
(86) International application number: PCT/EP2016/055081
(87) International publication number: WO 2016/146462

(56) References cited:
- US-A1- 2010 099 609
- MICHAEL WILLEM ET AL: "[eta]-Secretase processing of APP inhibits neuronal activity in the hippocampus", NATURE, vol. 526, no. 7573, 31 August 2015 (2015-08-31), pages 443-447, XP55269151, United Kingdom ISSN: 0028-0836, DOI: 10.1038/nature14864
- C. HAASS ET AL: "Trafficking and Proteolytic Processing of APP", COLD SPRING HARBOR PERSPECTIVES IN MEDICINE, vol. 2, no. 5, 7 February 2012 (2012-02-07), pages a006270-a006270, XP055202232, DOI: 10.1101/cshperspect.a006270
- L. J. VELLA ET AL: "Identification of a novel amyloid precursor protein processing pathway that generates secreted N-terminal fragments", THE FASEB JOURNAL, vol. 26, no. 7, 1 July 2012 (2012-07-01), pages 2930-2940, XP055202566, ISSN: 0892-6638, DOI: 10.1096/fj.11-200295
- J. BIEN ET AL: "The Metalloprotease Meprin Generates Amino Terminal-truncated Amyloid Peptide Species", JOURNAL OF BIOLOGICAL CHEMISTRY, vol. 287, no. 40, 28 September 2012 (2012-09-28), pages 33304-33313, XP055202580, ISSN: 0021-9258, DOI: 10.1074/jbc.M112.395608
- NIKOLAEV ANATOLY ET AL: "APP binds DR6 to trigger axon pruning and neuron death via distinct caspases", NATURE, NATURE PUBLISHING GROUP, UNITED KINGDOM, vol. 457, no. 7232, 1 February 2009 (2009-02-01), page 981, XP009145529, ISSN: 0028-0836, DOI: 10.1038/NATURE07767

## Description

The present invention relates to a peptide or collection of peptides derived from amyloid precursor protein (APP). The present invention also relates to uses of such peptide or collection of peptides, in particular as a diagnostic marker and/or as a drug target.

Intensive efforts are on the way to develop therapeutic strategies targeting Aβ, the major component of the Alzheimer's disease signifying amyloid plaques¹. Aβ is generated from APP by proteolytic processing. First, β-secretase, identified as BACE1, cleaves at the Met-Asp bond of the Aβ domain and generates CTF-β, which is further processed to Aβ by intramembrane cleavage by γ-secretase⁶. Liberated Aβ forms soluble synaptotoxic oligomers, which are believed to be the major culprit of the disease¹. Thus targeting the production of synaptotoxic Aβ species by β-secretase inhibition is a promising therapeutic strategy and clinical studies with high affinity BACE1 inhibitors are currently evaluated^{7,8}. Decreasing BACE1 activity leads to an increase of non-amyloidogenic processing via ADAM10⁹. Similarly, enhancing α-secretase activity reduced Aβ production and plaque formation¹⁰. However, stable isotope labeling kinetics upon *in vivo* inhibition of BACE1 in monkeys revealed an 83% decrease of sAPP-β but only a 35% increase of sAPP-α³. Thus, the fate of almost 50% of the initially labeled APP remains unclear. In addition to the two major and well-studied proteolytic processing pathways, APP is also shed in minor processing pathways utilizing different proteases¹¹. Furthermore, 17-35 kDa N-APP fragments are generated during early development and upon trophic-factor deprivation¹²⁻¹⁴. However, such alternative APP metabolites were not observed to accumulate upon BACE1 inhibition.

Hence, there is a need in the art to identify new pathways that are involved in the genesis of Alzheimer's disease. More specifically, there is a need in the art to identify molecular species which may either serve as a diagnostic marker or a drug target, more specifically, as a diagnostic marker or drug target for Alzheimer's disease.

In a first aspect, the present invention relates to a peptide or collection of peptides that is the reaction product of matrix-metalloproteinase MT1-MMP or MT5-MMP acting on amyloid precursor protein (APP), followed by the action of both α-secretase (ADAM10) and β-secretase (β-site APP cleaving enzyme 1; BACE1).

In one embodiment, the peptide or collection of peptides has one or several epitopes selected from MISEPRISYG (SEQ ID NO: 1), DALMPSLT (SEQ ID NO: 2), PWHSFGADSVP (SEQ ID NO: 3), SEVKM (SEQ ID NO: 4), and DAEFRHDSGYEVHHQK (SEQ ID NO: 5).

In one embodiment, the peptide or collection of peptides is characterized by recognition through an antibody which recognizes one or several of said aforementioned epitopes.

In one embodiment, the peptide or collection of peptides has a sequence selected from MISEPRISYGNDALMPSLTETKTTVELLPVNGEFSLDDLQPWHSFGADSVPANTENEV EPVDARPAADRGLTTRPGSGLTNIKTEEISEVKM (SEQ ID NO: 6) and MISEPRISYGNDALMPSLTETKTTVELLPVNGEFSLDDLQPWHSFGADSVPANTENEV EPVDARPAADRGLTTRPGSGLTNIKTEEISEVKMDAEFRHDSGYEVHHQK (SEQ ID NO: 7).

In one embodiment, one peptide has a sequence MISEPRISYGNDALMPSLTETKTTVELLPVNGEFSLDDLQPWHSFGADSVPANTENEVEPVDARPAADRGLTTRPGSGLTNIKTEEISEVKM (SEQ ID NO: 6) and another peptide has a sequence MISEPRISYGNDALMPSLTETKTTVELLPVNGEFSLDDLQPWHSFGADSVPANTENEVEPVDARPAADRGLTTRPGSGLTNIKTEEISEVKMDAEFRHDSGYEVHHQK (SEQ ID NO: 7).

In a further aspect, the present invention relates to a composition of peptides comprising one or several peptides according to the present invention and at least one further peptide that is the reaction product of matrix-metalloproteinase MT1-MMP or MT5-MMP only acting on amyloid precursor protein (APP).

In one embodiment, the at least one further peptide is membrane bound or is soluble in aqueous solution.

In one embodiment, the composition according to the present invention comprises both a membrane bound further peptide that is the reaction product of matrix-metalloproteinase MT1-MMP or MT5-MMP only acting on amyloid precursor protein (APP), and a peptide soluble in aqueous solution and being the reaction product of matrix-metalloproteinase MT1-MMP or MT5-MMP only acting on amyloid precursor protein (APP).

In one embodiment, said membrane bound further peptide has a sequence represented by SEQ ID NO: 8, and said peptide soluble in aqueous solution has a sequence represented by SEQ ID NO. 9.

The invention also relates to the peptide or collection of peptides or the composition of peptides according to the present invention, for use in a method of detecting Alzheimer's disease in a patient, wherein said method involves detecting said peptides or said collection of peptides.

In one embodiment, said use involves the detection of said peptide or of said collection or composition of peptides in cerebrospinal fluid (CSF) or plasma.

The present invention furthermore relates to the peptide or collection of peptides or the composition of peptides according to the present invention, for use in a method of treatment, prevention and/or alleviation of Alzheimer's disease.

In one embodiment, said method of treatment involves the inhibition in a patient, of formation or action of said peptide or said collection of peptides or of said composition of peptides, or the inhibition of action or production of matrix-metalloproteinase MT5-MMP and/or MT1-MMP.

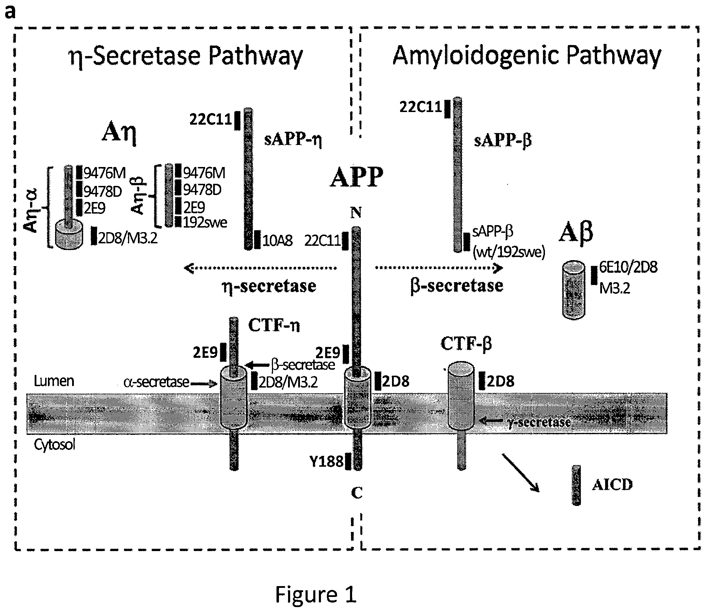

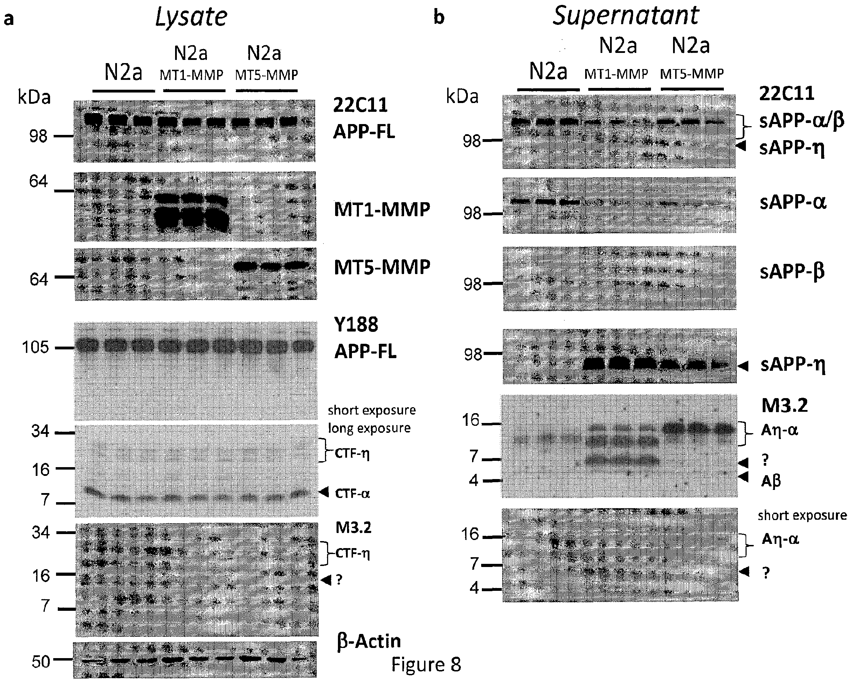

As used herein, a "peptide derived from amyloid precursor protein (APP) by the action of one or several matrix-metalloproteinases" is meant to refer to a peptide that is the reaction product of the one or several matrix-metalloproteinases acting on the amyloid precursor protein and cleaving the same into fragment(s). The present inventors have surprisingly identified a novel prominent proteolytic pathway which is initiated by the action of matrix-metalloproteinase 5 and/or matrix-metalloproteinase 1 or both. As used herein, the action of such matrix-metalloproteinase 5 and/or 1 is herein also sometimes referred to as "η-secretase". The present inventors believe that this novel proteolytic pathway is initiated by such η-secretase and is followed by the action of other secretases, most notably α-secretase (ADAM10) and/or β-secretase (β-site APP cleaving enzyme 1; BACE1). The resultant peptides are herein also sometimes referred to as "Aη-peptides" which comprise Aη-α and Aη-β (see also figure 1a and figure 8). These Aη-peptides turn out to inhibit hippocampal long-term potentiation (LTP), similar to the familiar Aβ-oligomers. Furthermore, the Aη-peptides accumulate upon genetic and pharmacological inhibition of β-secretase (BACE1). The peptides thus identified are useful as diagnostic markers and/or as drug targets. In particular, they are useful as diagnostic markers for Alzheimer's disease and as drug targets in the treatment, prevention and/or alleviation of Alzheimer's disease.

The present invention also relates to the use of the peptide(s) or collection of peptides according to the present invention for the manufacture of a medicament for the prevention, treatment and/or alleviation of Alzheimer's disease. In one embodiment, said use involves the administration of a compound to a patient, which compound inhibits the formation or action of one or several peptides according to the present invention. Preferably, said patient is a human patient.

The present invention also relates to a method of identifying a compound useful for the prevention, treatment and/or alleviation of Alzheimer's disease, wherein said compound is identified based on its capability to inhibit the formation or action of one or several peptides according to the present invention.

The present invention also relates to a method of detecting Alzheimer's disease in a patient, wherein said method involves detecting one or several peptides in accordance with the present invention, i. e. peptides, derived from amyloid precursor protein (APP) by the action of matrix-metalloproteinase MT1-MMP or MT5-MMP, and by the action of at least one enzyme selected from α-secretase (ADAM10) and β-secretase (β-site APP cleaving enzyme 1; BACE1). In one embodiment, said detection occurs by detecting said peptide(s) in cerebro-spinalfluid (CSF) and/or plasma. Preferably said patient is a human patient.

The present invention also relates to a method of treatment, prevention and/or alleviation of Alzheimer's disease, wherein said method involves the inhibition, in a patient, of the formation or the action of the peptide(s) in accordance with the present invention, or it involves the inhibition of the action or production of matrix-metalloproteinase MT5-MMP and/or MT1-MMP. In one embodiment, the inhibition of the formation or action of said peptide(s) is achieved by the administration to a patient of a drug that inhibits the formation of said peptide(s). In another embodiment, said method of treatment involves the administration to a patient of a drug that inhibits the action of said peptide(s). Inhibition of the formation of said peptide(s) may for example occur by binding to APP and/or to the enzyme(s) cleaving said APP. The inhibition of the action of said peptide(s) may occur by binding to said peptide(s) or to its (their) respective binding partner(s). Preferably, said patient is a human patient.

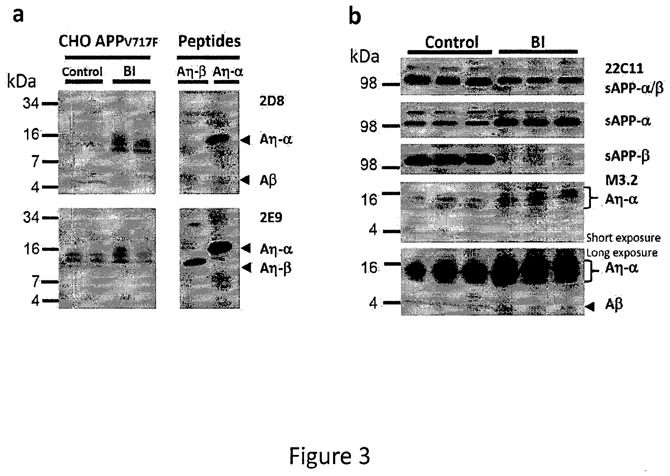

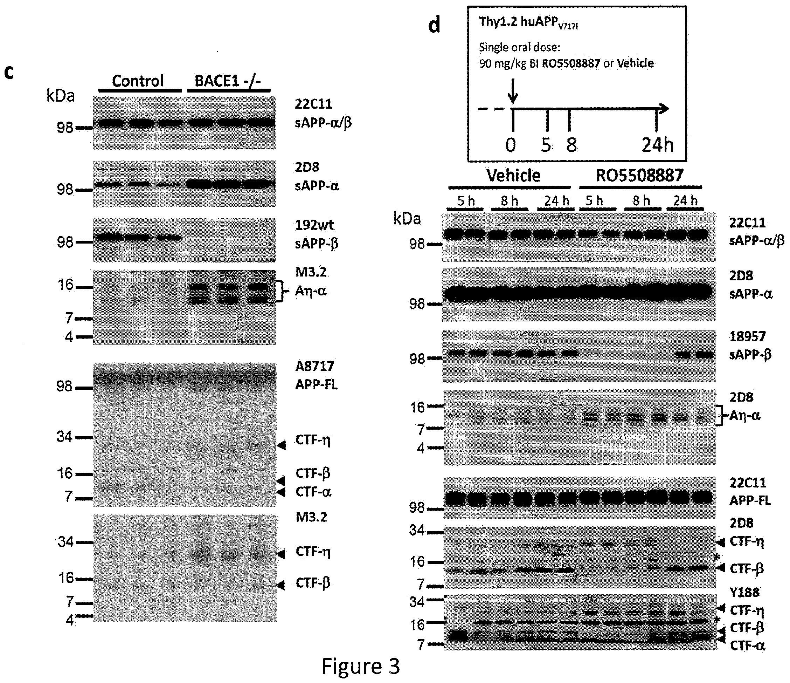

The specific sequences disclosed herein refer to and are derived from the specific splicing variant P05067-4 (as disclosed in the data base UniProt, human isoform APP695 of amyloid beta A4 protein). It should be clear, however, that the peptide(s) according to the present invention are not limited to the peptides derived from this specific splicing variant, and a person skilled in the art will be able to identify corresponding equivalent peptides from other splicing variants as well. It should also be noted that the sequences used herein are human sequences, despite the fact that some of the experiments performed herein were done with mice. However, the data obtained in figs. 3-4, 6-8 were obtained with human sequences, and there is an extremely high homology between the mouse and human sequences, lending further support to the general applicability of the present inventors' findings.

Furthermore, reference is made to the figures, wherein
Figure 1 presents data on the proteolytic processing pathway of APP in accordance with the present invention;
Figure 2 provides data on the physiological role of matrix-metalloproteinase MT5-MMP;
Figure 3 shows data with respect to the inhibition of β-secretase (BACE1);
Figure 4 shows data on the effects of peptide in accordance with the present invention, Aη-α and Aη-β on hippocampal long term potentiation (LTP);
Figure 5 shows a table on antibodies that were used in the experimental work leading to the present invention;
Figure 6 shows immunohistochemical data on dystrophic neurites in brains affected by Alzheimer's disease, using antibodies against All-epitope(s);
Figure 7 shows the effects of Aβ_{S26C}-dimers on long term potentiation (LTP).
Figure 8 shows the effects of overexpression of MT1-MMP and MT5-MMP in N2a cells.

### More specifically,

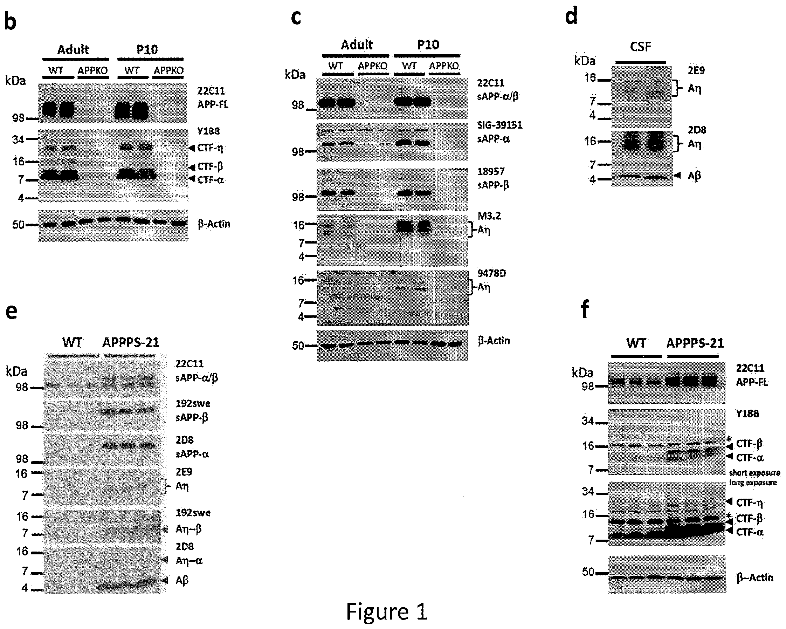

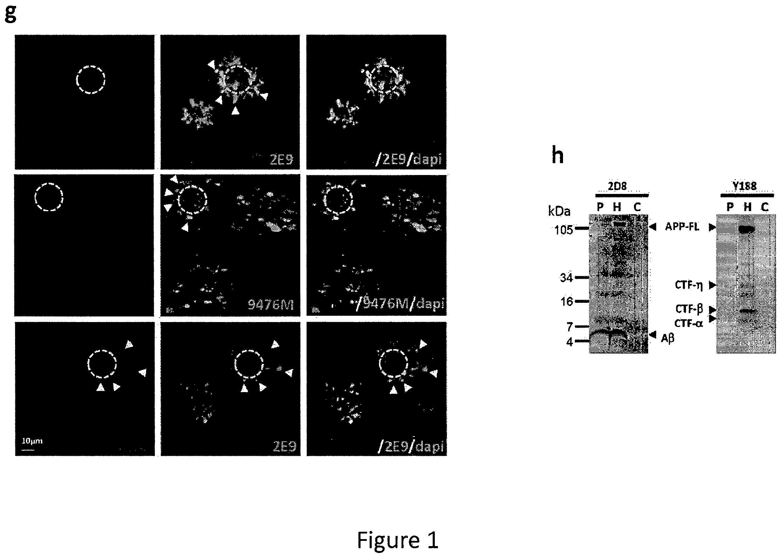

### Figure 1 shows a novel proteolytic processing pathway of APP and the data in respect thereof

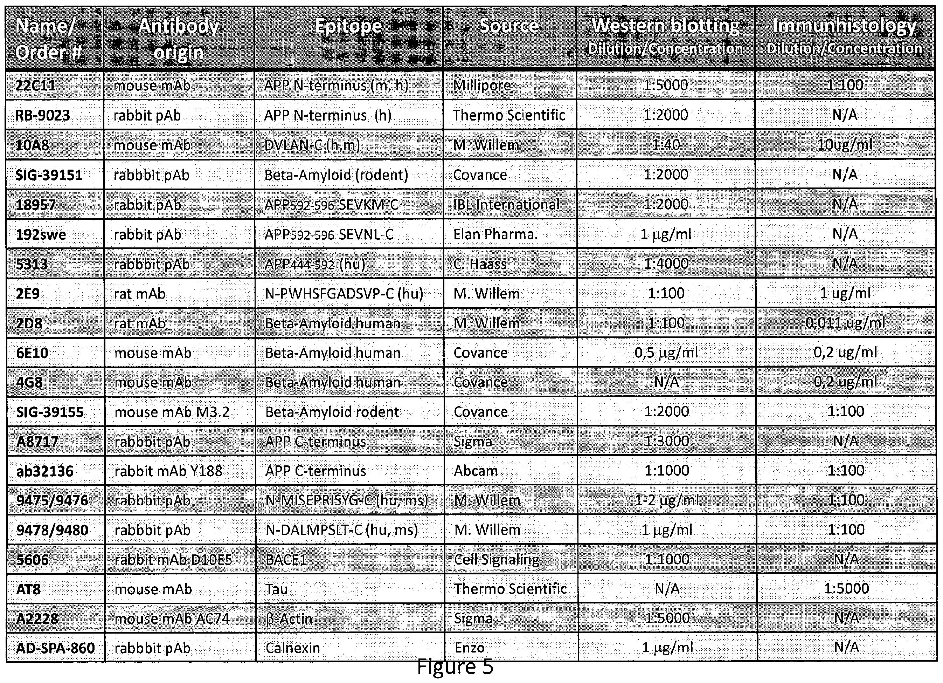

**a)** Schematic representation of the η-secretase pathway (left) and previously known amyloidogenic pathway (right). Antibodies used in this study are indicated (see also figure 5). **b)** A novel 30 kDa N-terminally elongated APP-CTF-η fragment is detected in membrane fractions obtained from brains of adult (22 month) and postnatal day 10 (P10) mice using the rabbit monoclonal antibody Y188 directed against the C-terminus of APP. CTF-η is specifically found in young and old wild type (WT) mice but absent in APP knockout mice (APPKO). In addition to this novel fragment, Y188 is detecting CTF-β and CTF-α. Full-length APP (APP-FL) was detected with antibody 22C11. β-Actin served as a loading control. c) Aη was identified as several closely spaced peptides detected in the soluble (DEA) fraction of adult age and P10 mice by antibody M3.2. A similar pattern is detected by antibody 9478D that is specifically recognizing an N-terminal part of the Aη peptide. Aη signal is more prominent in P10 mice compared to adult mice. Detection of sAPP-α and sAPP-β species is shown as additional control. APPKO brain were used as controls for antibody specificities. β-Actin served as a loading control. **d)** Aη and Aβ were readily detectable in 10 µl of human CSF by antibody 2D8 at similar intensities. The antibody 2E9 allowed the selective detection of Aη in the same samples, **e)** Soluble (DEA) extracts of APPPS-21 mouse brains contained Aη species detected by 2E9. Aη-β(swe) was selectively detected by antibody 192swe in addition to sAPP-β(swe). While 2D8 antibody detected robust levels of sAPP-α, only low levels of Aη-α could be detected in APPPS-21 brain lysates due to the overexpression of APPswe transgene. **f)** An increase in CTF-η is observed in RIPA lysates of APPPS-21 mouse brains (long exposure) using antibody Y188 as compared to WT. Full-length APP (APP-FL) was detected with antibody 22C11. β-Actin served as a loading control. **g)** Immunohistological stainings of cortical sections of 6 months old APPPS-21 transgenic mice revealed 6E10 positive Aβ plaque cores (encircled) surrounded by dystrophic neurites positive for 2E9 (white arrowheads, upper panel) and 9476M (white arrowheads, middle panel). Y188 (lower panel) staining co-localized with 2E9 positive signal (yellow arrowheads, lower panel). Nuclei were counterstained with DAPI. Calibration bar = 10 µm. **h)** Accumulation of CTF-η fragment in dystrophic neurites of 14 months old APPPS-21 mice. LCM of APPPS-21 brain sections bearing Thioflavin-S positive Aβ plaque core (P) and the surrounding Aβ plaque halo (H) was performed. As control (C) brain areas, devoid of plaques, were used. While Aβ was readily detected by antibody 2D8 in lysates containing plaque enriched material and halo regions (fractions P and H), CTF-η was selectively detected in the lysates prepared from the region enriched in dystrophic neurites (H), but not detected in plaque or control regions (P and C). As expected, CTF-β/α species are also enriched in dystrophic neurites (H).

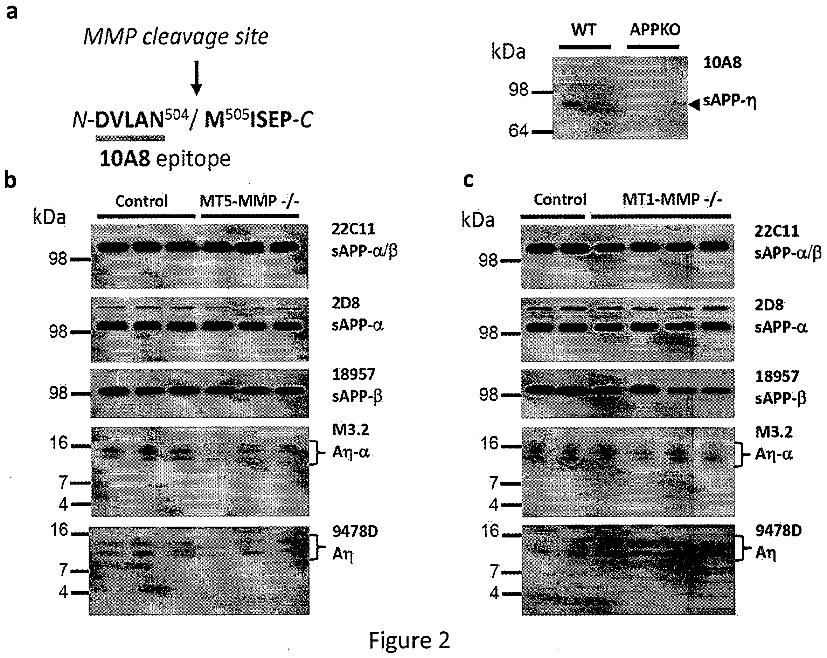

### Figure 2 shows that MT5-MMP has physiological η-secretase activity in brain

**a)** MMPs can cleave human APP₆₉₅ at the indicated position (arrow) between amino acid N504 and M505 in the N-terminal domain. The epitope for the novel cleavage-specific antibody 10A8 is indicated (grey). sAPP-η of 80 kDa was specifically detected in DEA extracts of P10 WT mouse brain using the antibody 10A8, but was absent in APP KO brains. **b-c)** Soluble Aη levels, detected by the 9478D and M3.2 antibodies (Aη-α) were reduced in MT5-MMP -/- mouse brains, but unchanged in MT1-MMP -/- brains. Soluble (DEA) brain lysates were prepared from P10 mice. Total levels of secreted APP (22C11), sAPP-α or sAPP-β were unchanged.

### Figure 3 shows that an inhibition of BACE1 results in elevated levels of CTF-η and of Aη-α

**a**) Supernatants of CHO cells expressing human APP_{V717F} without or with BACE1 inhibition (BI; 2 µM Merck IV) were compared to synthetic peptides of Aη-β and Aη-α. Increased Aη-α peptide levels were observed upon BACE1 inhibitor treatment with 2D8 and 2E9 antibodies. The fragment with the lowest molecular weight, co-migrating with the synthetic peptide Aη-β, disappeared upon BACE1 inhibition **b)** After overnight incubation of DIV16 primary hippocampal neurons without or with the BACE1 inhibitor (BI; 2 µM Merck IV), supernatants were analyzed by Western blotting. A strong increase of endogenous Aη-α was detected with M3.2 antibody. Total levels of secreted APP (22C11) were unchanged while sAPP-α levels increased. The absence of sAPP-β and Aβ proves the effective blockade of BACE1. **c)** Western blot analysis of DEA extracts of P10 BACE1 -/- mouse brains revealed a significant increase in Aη-α peptides as compared to controls. Total levels of secreted APP (22C11) were unchanged while sAPP-α levels increased due to compensation by α-secretase activity. CTF-η levels were increased in RIPA lysates of the BACE1 KO mouse brain. As expected after an efficient BACE1 block, CTF-β and sAPP-β were severely reduced. **d**) BACE1 inhibition *in vivo* resulted in enhanced production of Aη-α species. A schematic presentation of the study design with a single oral dose treatment of APP_{V717I} mice with the BACE1 inhibitor RO5508887 is shown in the upper panel. Inhibitor treated mice and vehicle treated controls were sacrificed and analyzed after 5, 8 or 24 h. BACE1 inhibition reduced sAPP-β and CTF-β and increased levels of Aη-α at 5 and 8 h after treatment. 24 h after the treatment these changes were normalized due to the clearance of the inhibitor (a background band obtained with Y188 is indicated by asterisk).

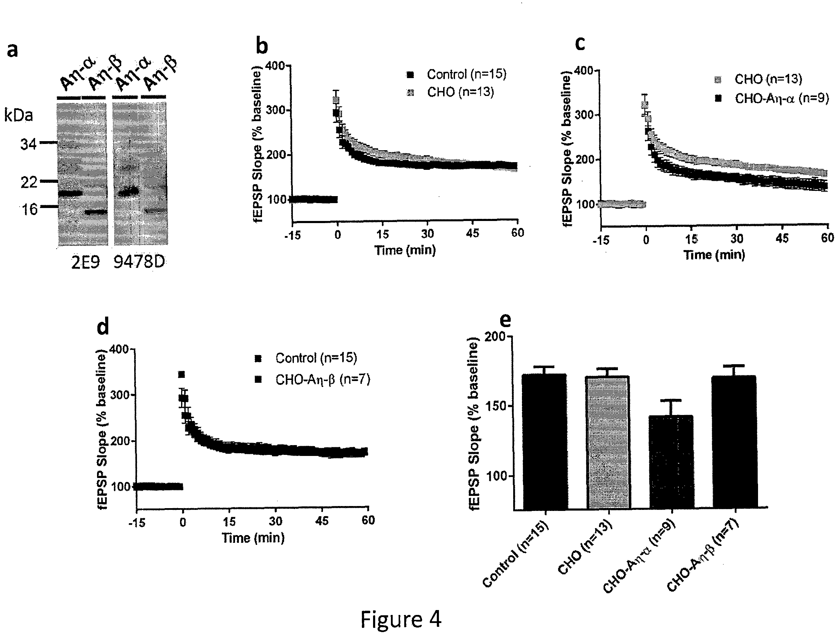

### Figure 4 shows that Aη-α impairs hippocampal LTP.

a) Aη-α and Aη-β peptides were expressed in CHO cells and collected in OPTIMEM medium. Western blot analysis revealed the larger Aη-α and the smaller Aη-β peptides. Higher molecular weight bands are most likely due to posttranslational modification by glycosylation. **b-e)** SEC fractions enriched for Aη were diluted (1:15) in ACSF for the treatment of hippocampal slices and LTP measurements. Aη-α, Aη-β and control SEC fractions (obtained from CHO cells transfected with the empty vector) were perfused over mouse hippocampal slices for 15 min after obtaining a stable baseline of a fEPSP at the CA3-CA1 synapse. At the end of these 15 minutes, a high-frequency stimulation protocol was applied (HFS; 2x (100 Hz, 1 s) at 20 second inter-stimulus interval) to induce long-term potentiation (LTP). **b)** CHO supernatant by itself did not alter LTP when compared to ACSF control. **c)** SEC fractions from conditioned media of CHO cells expressing Aη-α significantly inhibited LTP. **d)** SEC fractions from conditioned media of CHO cells expressing Aη-β did not significantly inhibit LTP. **e)** Quantification of LTP magnitudes (as % of baseline) calculated 45-60 minutes post-HFS from graphs in b-d with statistical analysis (*p < 0.05); error bars represent s.e.m. n= number of fields.

### Figure 5 shows a table of antibodies used in this study.

List of antibodies used with indicated specificity, antibody epitope (if known) and dilutions applied in Western blotting and immunostaining techniques.

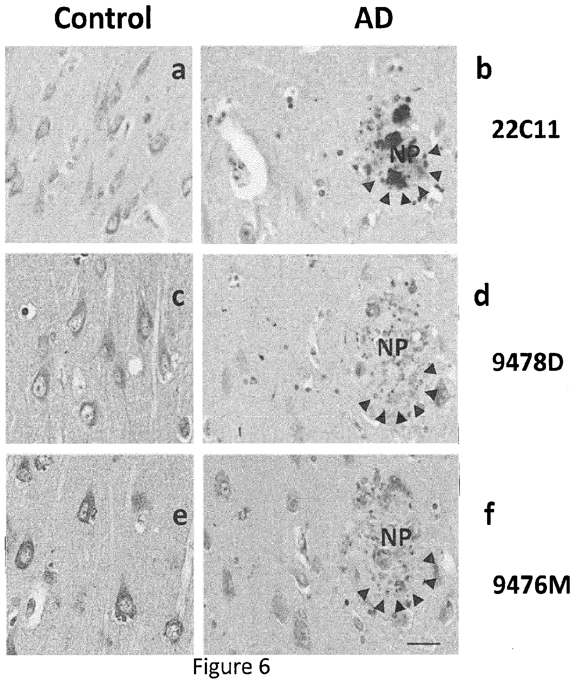

### Figure 6 shows that dystrophic neurites in AD brains are positive for Aη-epitope antibodies.

Immunohistochemistry with 22C11 (**a, b**), 9478D (**c, d**) and 9476M (**e, f**) in the human hippocampus (CA1-subiculum region) of a control case (**a, c, e**) and in the AD case (**b, d, f**). Immuno-positive signals were observed with 22C11 (**a**), 9478D (**c**) and 9476M (**e**) in the so-mata and neuropils of normal and AD brain. In AD brains these antibodies decorate dystrophic neurites (**b, d, f**). Calibration bar = 30 µm.

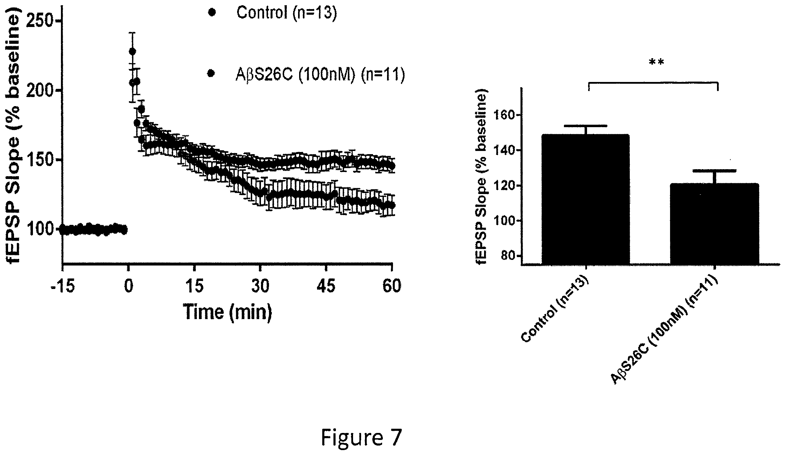

### Figure 7 shows that Aβ_{S26C} dimers impair hippocampal LTP.

a) Treatment with Aβ_{S26C} dimers (100 nM final; JPT Peptide Technologies, Germany; diluted in 25 ml re-circulating ACSF) reduced LTP as compared to interleaved LTP recordings in 25 ml re-circulating ACSF. b) Illustrated is the average LTP magnitude (at 45-60 minutes post-LTP induction) normalized to pre-LTP baseline values (100%) in untreated conditions (**p<0.01).

### Figure 8 shows high levels of Aη-α upon overexpression of MT1-MMP and MT5-MMP in N2a cells.

a, Expression of MT1-MMP and MT5-MMP in stably transfected N2a cells were analyzed in lysates with specific antibodies. No major difference was observed in the levels of APP-FL (APP full length) and APP-CTFs, while 2 predominant bands were observed in the range of 20-30 kDa. With antibodies Y188 and with M3.2 an accumulation of CTF-η was detected in both cell lines. While the smaller CTF-η species was enriched in N2a MT1-MMP cells, the higher CTF-η species was enriched in N2a MT5-MMP cells. In lysates of N2a MT1-MMP cells additionally a 16 kDa fragment was identified by both antibodies and as APP-CTF derived from a cleavage closer to the β-secretase cleavage site. **b,** In supernatants of N2a cells expressing either MT1-MMP or MT5-MMP the levels of sAPP-α and sAPP-β were substantially reduced, while sAPP-η was strongly increased as detected with the antibody 10A8. The antibody M3.2, directed against the murine N-terminal Aβ domain, allowed to specifically detect a longer Aη-α species of 16 kDa in the supernatants of N2a MT5-MMP cells, while a slightly shorter Aη-α species of 14 kDa was observed in the supernatants of N2a MT1-MMP cells. Here additionally a 7 kDa species was detected with the antibody M3.2. While this peptide is most likely derived from the 16 kDa CTF observed in the N2a MT1-MMP lysates, the Aη-α species of higher or lower molecular weight correspond to the species seen in control N2a supernatants and fit to the different sizes found for the CTF-Aη species in the respective cell lysates.

Furthermore, reference is made to the following sequences which are given as exemplary embodiments:
All sequences are Homo sapiens sequences. It should be noted that the sequences indicated in the present application are denoted such that their N-terminal end is at the left side, and their C-terminal end is at the right side. Hence, as an example, the sequence MISEPRISYG has the N-terminal end at M and the C-terminal end at G.
**SEQ ID NO:1:**
   MISEPRISYG
   Epitope on Aη-peptide(s)
**SEQ ID NO:2:**
   DALMPSLT
   Epitope on Aη-peptide(s)
**SEQ ID NO:3:**
   PWHSFGADSVP
   Epitope on Aη-peptide(s)
**SEQ ID NO:4:**
   SEVKM
   Epitope on Aη-peptide(s)
**SEQ ID NO:5:**
   DAEFRHDSGYEVHHQK
   Epitope on Aη-peptide(s)
**SEQ ID NO:6:**

### Example of an Aη-β-peptide (residues 505-596 of P05067-4)

**SEQ ID NO:7:**

### Example of an Aη-α-peptide (residues 505-612 of P05067-4)

**SEQ ID NO:8:**

### Example of an CTF-η-peptide (residues 505-695 of P05067-4)

**SEQ ID NO:9:**

### Example of an sAPPη-peptide (residues 19-504 of P05067-4)

**SEQ ID NO:10:**

### Human isoform APP695 of amyloid beta A4 protein (P05067-4)(residues 1-695)

The term "antibody," as used herein, refers to an immunoglobulin molecule which is able to specifically bind to a specific epitope on an antigen. Antibodies can be intact immunoglobulins derived from natural sources or from recombinant sources and can be immunoreactive portions of intact immunoglobulins. Antibodies are typically tetramers of immunoglobulin molecules. The antibodies used in the context of the present invention may exist in a variety of forms including, for example, polyclonal antibodies, monoclonal antibodies, Fv, Fab and F(ab)₂, as well as single chain antibodies and humanized antibodies (Harlow et al., 1999, Using Antibodies: A Laboratory Manual, Cold Spring Harbor Laboratory Press, NY; Harlow et al., 1989, Antibodies: A Laboratory Manual, Cold Spring Harbor, New York; Houston et al., 1988, Proc. Natl. Acad. Sci. USA 85:5879-5883; Bird et al., 1988, Science 242:423-426).

Antibodies may be generated using recombinant DNA technology, such as, for example, an antibody expressed by a bacteriophage as described herein. The term "antibody" should also be construed to mean an antibody which has been generated by the synthesis of a DNA molecule encoding the antibody and which DNA molecule expresses an antibody peptide, or an amino acid sequence specifying the antibody, wherein the DNA or amino acid sequence has been obtained using synthetic DNA or amino acid sequence technology which is available and well known in the art.

Monoclonal antibodies directed against full length or peptide fragments of a peptide or peptide may be prepared using any well known monoclonal antibody preparation procedures, such as those described, for example, in Harlow et al. (1988, In: Antibodies, A Laboratory Manual, Cold Spring Harbor, NY) and in Tuszynski et al. (1988, Blood, 72:109-115). Quantities of the desired peptide may also be synthesized using chemical synthesis technology. Alternatively, DNA encoding the desired peptide may be cloned and expressed from an appropriate promoter sequence in cells suitable for the generation of large quantities of peptide. Monoclonal antibodies directed against the peptide are generated from mice or other animals, such as rabbits, hamsters, etc. immunized with the peptide using standard procedures as referenced herein.

Nucleic acid encoding the monoclonal antibody obtained using the procedures described herein may be cloned and sequenced using technology which is available in the art, and is described, for example, in Wright et al. (1992, Critical Rev. in Immunol. 12(3,4):125-168) and the references cited therein. Further, the antibody of the invention may be "humanized" using the technology described in Wright et al., (*supra*) and in the references cited therein, and in Gu et al. (1997, Thrombosis and Hematocyst 77(4):755-759).

To generate a phage antibody library, a cDNA library is first obtained from mRNA which is isolated from cells, e.g., the hybridoma, which express the desired peptide to be expressed on the phage surface, e.g., the desired antibody. cDNA copies of the mRNA are produced using reverse transcriptase. cDNA which specifies immunoglobulin fragments are obtained by PCR and the resulting DNA is cloned into a suitable bacteriophage vector to generate a bacteriophage DNA library comprising DNA specifying immunoglobulin genes. The procedures for making a bacteriophage library comprising heterologous DNA are well known in the art and are described, for example, in Sambrook and Russell (2001, Molecular Cloning: A Laboratory Manual, Cold Spring Harbor, NY).

Bacteriophage which encode the desired antibody, may be engineered such that the peptide is displayed on the surface thereof in such a manner that it is available for binding to its corresponding binding peptide, e.g., the antigen against which the antibody is directed. Thus, when bacteriophage which express a specific antibody are incubated in the presence of a cell which expresses the corresponding antigen, the bacteriophage will bind to the cell. Bacteriophage which do not express the antibody will not bind to the cell. Such panning techniques are well known in the art and are described for example, in Wright et al., (supra).

Processes such as those described above, have been developed for the production of human antibodies using M13 bacteriophage display (Burton et al., 1994, Adv. Immunol. 57:191-280). Essentially, a cDNA library is generated from mRNA obtained from a population of antibody-producing cells. The mRNA encodes rearranged immunoglobulin genes and thus, the cDNA encodes the same. Amplified cDNA is cloned into M13 expression vectors creating a library of phage which express human antibody fragments on their surface. Phage which display the antibody of interest are selected by antigen binding and are propagated in bacteria to produce soluble human immunoglobulin. Thus, in contrast to conventional monoclonal antibody synthesis, this procedure immortalizes DNA encoding human immunoglobulin rather than cells which express human immunoglobulin.

Moreover, reference is made to the following examples which are given to illustrate, not to limit the present invention:

### Examples

### Example 1

### Material and methods

### Biochemical methods

Soluble proteins were extracted from brain hemispheres with DEA buffer³⁹, membrane proteins were extracted with RIPA buffer or by applying a membrane preparation protocol as described⁴⁰. All Western blot procedures were done essentially as described⁴¹.

### BACE1 inhibitor treatment

Three-month-old heterozygous female transgenic mice FVB/N x C57B1/6J expressing APP_{V717I}²⁸ were used for BACE1 inhibition studies. Gavage mediated administration of BACE1 inhibitor RO5508887 (90 mg/kg, 14.06 ml/kg) or vehicle (14.06 ml/kg) was performed once²⁷.

### Slice preparation and electrophysiological recordings

OPTIMEM was used to collect CHO cell supernatants. SEC was performed with concentrated CHO supernatants using a FPLC (ÄKTApurifier) equipped with a Superdex75 column (GE Healthcare). 1 ml samples were collected with a mobile phase flow rate set at 0.5 ml/min in standard ACSF. Acute hippocampal slices were prepared from Swiss mice (PND20-30) and kept in ACSF⁴². Extracellular field excitatory post-synaptic potentials (fEPSPs) were obtained from CA1 pyramidal neurons. After 15 minutes of bath application of SEC fractions⁴³, LTP was induced by high frequency stimulation (2 pulses of 100Hz for 1 second with a 20 second inter-pulse interval). LTP was recorded for 60 minutes and statistical analysis was performed on the last 15 minutes of recording compared to baseline fEPSP values.

### Cell culture

CHO and 7PA2⁴⁴ cells were grown in DMEM/F12 (Thermo Scientific) supplemented with 10% fetal calf serum (FCS, Thermo Scientific) plus Penicillin/Streptomycin and NEAA (Non-essential amino acids, PAA) in a humid incubator with 5% CO₂ at a temperature of 37°C. For inhibitor treatment, cell culture medium was replaced with fresh, pre-warmed serum free medium (OPTIMEM; Thermo Scientific) supplemented with inhibitors or DMSO as vehicle control. Treatment was initiated when cells reached 90-100% confluency and conditioned media were harvested after 20-24 h. Supernatants were cleared by centrifugation (10', 5500 g at 4°C). To obtain cell lysates, cell monolayers were washed once with ice-cold PBS and detached in 1 ml PBS using a cell scraper. The cell suspension was pelleted by centrifugation (5', 1000 g at 4°C) and lysed with RIPA buffer (20 mM sodium citrate pH 6.4, 1 mM EDTA, 1% Triton X-100 in ddH₂O) supplemented with Protease Inhibitor Cocktail (Sigma-Aldrich). The protein concentration of lysates was determined using the Uptima BC Assay Protein Quantitation kit (Interchim).

### Primary cell culture

Hippocampal neurons were isolated from embryonic day 18 CD rats (Charles River) as described⁴⁵. Dissociated neurons were plated at 17,700 cells per cm² onto 6 cm dishes coated with poly-L-lysine (1 mg/mL; Sigma) and cultured in Neurobasal medium supplemented with 2% B27 and 0.5 mM L-glutamine (all from Invitrogen). Hippocampal cultures were maintained in a humidified 5% CO₂ incubator at 37°C. For inhibitor treatment, DIV16 culture medium was replaced with fresh, pre-equilibrated N2 medium (supplemented with 20% of four days conditioned N2 medium from pure primary cultured astrocytes) to which inhibitors or DMSO as vehicle control were added⁴⁵.

### Transgenic mice, animal care, and animal handling

BACE1 -/- and APPPS-21 mice were described before^{46,47} and were bred for this study in a B16C57/J background. All treatments were approved by the local committee for animal use and were performed in accordance to state and federal regulations (license number KVR-1/221-TA116/09). Mice had access to pre-filtered sterile water and standard mouse chow (Ssniff® Ms-H, Ssniff Spezialdiäten GmbH, Soest, Germany) ad libitum and were housed under a reversed day-night rhythm in IVC System Typ II L-cages (528 cm²) equipped with solid floors and a layer of bedding, in accordance to local legislation on animal welfare.

### BACE1 inhibitor treatment

APP_{V717I}⁴⁸ mice were treated with the inhibitor RO5508887 provided by Hoffmann-La Roche ⁴⁹. Three-month-old heterozygous female transgenic mice in mixed FVB/N x C57B1/6J background expressing hAPP_{V717I}⁴⁸ were used for BACE1 inhibition studies. Gavage mediated administration of BACE1 inhibitor (90 mg/kg, 14.06 ml/kg) or vehicle (14.06 ml/kg) was performed once⁴⁹. The BACE1 inhibitor was diluted in 5% ethanol (Merck) and 10% solutol (Sigma-Aldrich) in sterile water (Baxter). Animals were sacrificed after 5, 8 and 24 h. Mice were anesthetized with 3.5 µl per gram body weight of a mixture of ketamine (115 mg/ml ketamine hydrochloride, Eurovet), xylazin 2% (23.32 mg/ml xylazine hydrochloride, VMD Arendonk), atropine (0.50 mg/ml atropine sulphate, Sterop) and saline (8:5:2:5, v/v/v/v). For brain preparation, mice were flushed trans-cardially with ice-cold saline (3.5 ml/min, 3 min). The brain was removed from the cranium and dissected into left and right hemiforebrain, brainstem, cerebellum and olfactory bulb. The brain structures were promptly immersed in liquid nitrogen and stored at -80 °C. Different tissues (kidneys, spleen, liver, stomach, gut, lungs and heart) were examined and checked for gross abnormalities. No obvious abnormalities were observed in any of the treatment groups.

### Preparation of protein extracts from brain

Brains were removed from the cranium and dissected into left and right hemispheres. Brain tissue was snap frozen in liquid nitrogen and stored at -80 °C. Soluble proteins were extracted with DEA buffer (50 mM NaCl, 0,2 % Diethylamine, pH 10 + protease inhibitor (P8340, Sigma-Aldrich)⁵⁰, membrane proteins were extracted with RIPA buffer (20 mM Tris-HCl (pH 7.5), 150 mM NaCl, 1 mM EDTA, 1 mM EGTA,1% NP-40,1% sodium deoxycholate, 2.5 mM sodium pyrophosphate + protease inhibitor) or applying a membrane preparation protocol as described before⁵¹.

### Protein analysis

Proteins were separated under denaturing conditions using discontinuous SDS-PAGE. Equal amounts of proteins denatured in Laemmli buffer were loaded onto the gel and 10 µl of the SeeBlue Plus2 Prestained Standard (Invitrogen) served as molecular weight marker. Electrophoresis was performed in Tris-glycine buffer (25 mM Tris, 190 mM glycine in ddH₂O) using the Mini-PROTEAN system (BIORAD) on activated PVDF membranes. Low molecular weight proteins (< 16 kDa) were separated using precast gradient Tricine Protein Gels (10-20%, 1 mm, Novex) in Tris-tricine buffer using the XCell SureLock Mini-Cell system (Novex). After separation by SDS-PAGE proteins were transferred onto membranes using the tank/wet Mini Trans-Blot cell system (BIORAD). CTFs, Aη and Aβ were detected after transfer on Nitrocellulose membranes (Protran BA85; GE Healthcare), while other proteins were blotted on PVDF (Immobilon-P, Merck Millipore). As size markers for Aη synthetic peptides Aη-β (92 aa; MISEPRISYGNDALMPSLTETKTTVELLPVNGEFSLDDLQPWHSFGADSVPANTENEV EPVDARPAADRGLTTRPGSGLTNIKTEEISEVKM⁹²) (= SEQ ID NO: 6) and the slightly longer Aη-α (108 aa; MISEPRISYGNDALMPSLTETKTTVELLPVNGEFSLDDLQPWHSFGADSVPANTENEV EPVDARPAADRGLTTRPGSGLTNIKTEEISEVKMDAEFRHDSGYEVHHQK¹⁰⁸) (= SEQ ID NO: 7) were obtained from Peptide Specialty Laboratories (PSL; Heidelberg, Germany). Upon completion of the transfer and prior to blocking, proteins transferred to nitrocellulose membranes were additionally denatured by boiling the membrane in PBS (140 mM NaCl, 10 mM Na₂HPO₄, 1.75 mM KH₂PO₄, 2.7 mM KCl in ddH2O, pH 7.4) for 5 min. After cooling to room temperature nitrocellulose membranes as well as the PVDF membranes were blocked in I-Block solution (0.2% Tropix I-Block (Applied Biosystems), 0.1% Tween20 in PBS) for 1 h at room temperature or o/n at 4°C (with agitation). Transferred proteins were detected using immunodetection and enhanced chemiluminescence (ECL). First, blocked membranes were incubated with primary antibodies diluted in I-Block solution o/n at 4°C (with agitation). After removal of the antibody, membranes were washed 3x in TBS-T buffer (10 min each, at room temperature, with agitation; 140 mM NaCl, 2.68 mM KCl, 24.76 mM Tris, 0.3% Triton X-100 in ddH₂O, pH 7.6) and subsequently incubated with a horseradish peroxidase (HRP) coupled secondary antibody (obtained from Promega or Santa Cruz). Secondary antibodies were diluted in I-Block solution and membranes were incubated for 1 h at RT (with agitation) followed by three washes in TBS-T. For ECL detection, membranes were incubated with HRP substrate (ECL, GE Healthcare or ECL Plus, Thermo Scientific) for 1 min at RT and signals were captured with X-ray films (Super RX Medical X-Ray, Fujifilm), which were subsequently developed using an automated film developer (CAWOMAT 2000 IR, CAWO).

### Human CSF samples

Human CSF samples were collected at the Dept. of Neurology Outpatient unit for neurodegenerative disease (KBFZ) of the University of Bonn. CSF was obtained by lumbar puncture at position L3, centrifuged and divided in small aliquots. For further analysis, samples were stored at -80°C. Turbid or blood contaminated samples were excluded from analysis. Use of these samples for research purposes has been consented by all patients according to the ethical committee requirements of the University of Bonn Ethical committee and approval number 279/10.

### Neuropathology & Immunohistochemistry

Braak-NFT stages⁵², and CERAD⁵³ scores for neuritic plaques were used to determine the degree of AD pathology according to the NIA-AA guidelines⁵⁴. Consecutive paraffin sections from the human medial lobe were stained with 22C11, 9476M and 9478D Primary antibodies were detected with biotinylated anti-mouse and anti-rabbit IgG secondary antibodies and visualized with avidin-biotin-complex (ABC-Kit, Vector Laboratories) and Diaminobenzidine-HCl (DAB). The sections were counterstained with Haematoxylin. Positive and negative controls were performed. 9476M and 9478D stainings were assessed in 10 control and 10 AD patient cases.

For double immunofluorescence analysis of APPPS-21 brain sections, 6 month old mice were sacrificed by CO₂ inhalation according to animal handling laws. Brains were dissected and fixed with 4% paraformaldehyde (PFA) in 0.1M PBS, pH 7.4 for 48 hours. For immunohistochemistry, 25 µm-thick sagittal mouse brain cryosections were treated with 10 mM sodium citrate, pH 6 at 93°C for 20 minutes, washed with 0,5% Triton X-100 in PBS, blocked with 5% goat serum (Invitrogen) and 0,5% Triton X-100 in PBS for 1 h and subsequently incubated overnight with primary antibodies diluted in blocking solution. Primary antibodies were used as listed in **figure 5**. DAPI was used to counterstain nuclei. Signals were visualized using fluorescently labeled secondary antibodies. Confocal images were acquired using a Plan-Apochromat 25x/0.8 oil differential interference contrast (DIC) objective on a LSM 710 confocal microscope (Zeiss) in sequential scanning mode using ZEN 2011 software package (black edition, Zeiss).

### Laser Capture Microdissection (LCM)

For laser capture microdissection of plaque cores and halos, 14 months old transgenic APPPS-21 mice were used according to a previously published protocol⁵⁵ with slight modifications. Mice brains were dissected, embedded in Shandon M1 embedding matrix (Thermo Scientific) and immediately frozen on crushed dry ice. 10 µm-thick sagittal sections were cut using a Microm HM 560 cryostat (Thermo Scientific), mounted on frame slides containing a 1.4 µm polyethylene terephthalate (PET)-membrane (Leica Microsystems) and subsequently stained or stored at -80°C for later usage. Staining was performed as follows: brain sections were thawed shortly at room temperature, fixed with 75% ethanol for 1 min, stained with 0.05% Thioflavin-S for 5 min, washed with 75% ethanol and dried at room temperature. LCM was performed on the same day using a laser dissection microscope (Leica, LMD 7000) with the following settings: excitation wavelength 495 nm, laser power 30, aperture 5, speed 6 and pulse frequency 119. Plaque cores and halos were cut using a 63x magnification objective and control non-plaque areas using a 10x magnification objective and collected in 0.5 ml caps (Leica Microsystem) for protein analysis. Lysates were done essentially as described above using RIPA with 0,1% SDS.

### Slice preparation and electrophysiological recordings

Transverse hippocampal slices (350 µm) were prepared from postnatal day 20-30 Swiss mice following standard procedures⁵⁶. Slices were cut in ice-cold oxygenated (95% O2/5% CO₂) solution containing 206 mM sucrose, 2.8 mM KCl, 1.25 mM NaH₂PO₄, 2 mM MgSO₄, 1 mM MgCl₂, 1 mM CaCl₂, 26 mM NaHCO₃, 0.4 mM sodium ascorbate, 10 mM glucose (pH 7.4). For recovery (1 h), slices were incubated at 27°C in oxygenated standard artificial cerebrospinal fluid (ACSF) containing: 124 mM NaCl, 2.8 mM KCl, 1.25 mM NaH₂PO4, 2 mM MgSO₄, 3.6 mM CaCl₂, 26 mM NaHCO₃, 0.4 mM sodium ascorbate, 10 mM glucose (pH 7.4)⁵⁷. Slices were inspected in a chamber on an upright microscope (Slicescope, Scientifica Ltd) with IR-DIC illumination and were perfused with the oxygenated ACSF at 27 ± 1 °C. Field excitatory post-synaptic potentials (fEPSPs) were recorded in the stratum radiatum of the CA1 region using a glass electrode (filled with 1 M NaCl/10mM HEPES, pH 7.4) and the stimuli (30% of maximal fEPSP) were delivered to the Schaeffer Collateral pathway by a monopolar glass electrode (filled with ACSF). Electrodes were specifically placed just below the surface of the slice to maximize the exposure to circulating peptides. A minimum of 15 minutes stable baseline was first obtained in standard ACSF followed by another 15 minutes of bath application of ACSF containing SEC fractions (CHO, Aη-α or Aη-β; 1/15 dilution, interleaved recordings) using re-circulation with a peristaltic pump at 2.5-3 ml min⁻¹ while being continuously aerated with 95% oxygen. No alterations in fEPSP baseline responses were observed after incubation with the SEC fractions. In the continuous presence of the the ACSF/SEC solution, LTP was then induced using a high frequency stimulation (HSF) protocol with 2 pulses of 100 Hz for 1 sec with 20 sec interval between pulses, and recorded for one hour. Control recordings (no application of SEC fractions) were obtained in an interleaved fashion where ACSF was re-circulated using an identical procedure. For LTP analysis, the first third of the fEPSP slope was calculated in baseline condition (15 minutes prior to induction of LTP and for 60 minutes post-induction). The average baseline value was normalized to 100% and all values of the experiment were normalized to this baseline average (one minute bins). Experiments were pooled per condition and presented as mean±s.e.m. Data analysis was performed with the clampfit software (Molecular devices). Statistical analysis was performed using GraphPad (Prism 6) on the last fifteen minutes of the recordings vs the 15 minutes of baseline using a one way-ANOVA and post-hoc Bonferroni test.

### Example 2

### Results

To identify novel proteolytic processing pathways of APP, the present inventors searched for C-terminal fragments (CTFs) of APP different from those giving rise to p3 (CTF-α) or Aβ (CTF-β) in membrane fractions of mouse brains¹⁵⁻¹⁷. Indeed, this revealed a novel CTF with an approximate molecular weight of 30 kDa, recognized by an antibody to the C-terminus of APP (Y188), which is absent in the brains of APP knockout mice (APPKO)¹⁸ (**Fig. 1b****;** antibodies used are described in **the table in** **figure 5**). The molecular weight of the novel CTF suggests a physiological cleavage of APP N-terminal to the known shedding sites of β-, and α-secretases, which the inventors named in analogy η-cleavage (**Fig. 1a**). In the soluble fraction, the present inventors detected the N-terminal cleavage product (sAPP-η; see **Fig. 2a**), whose molecular weight of approximately 80 kDa clearly distinguishes it from alternative N-terminal APP fragments described previously¹²⁻¹⁴. In addition, the present inventors observed lower molecular weight soluble fragments (Aη), which may derive from BACE1 (Aη-β) or ADAM10 (Aη-α) mediated cleavage of CTF-η (**Fig. 1a**). Aη was identified in the soluble fraction as several closely spaced peptides by antibody M3.2 (**Fig. 1c****),** demonstrating that some of these fragments contain the N-terminal part of the Aβ domain and are most likely ending at the α-secretase cleavage site (see also **Figures 1e** and **3a**). Aη fragments were further validated by antibody 9478D directed against an epitope N-terminal to the Aβ domain (**Fig. 1c****).** The presence of endogenous CTF-η as well as Aη in brains of mice suggests that Aη generation is a physiological processing pathway similar to Aβ production^{19,20}. To provide further evidence for Aη production in human brain, the present inventors analyzed cerebrospinal fluid (CSF). Robust signals of Aη were detected with the novel antibody 2E9 (epitope was identified as indicated in **figure 5**) confirming physiological Aη production *in vivo* in humans (**Fig. 1d**). In addition to the physiological production of Aη in wild type mice and human CSF, the present inventors observed increased Aη synthesis in soluble fractions of APPPS-21 transgenic mice²¹ as compared to wild type mice (**Fig 1e**). This allowed to identify the Aη-β species terminating at the BACE1 cleavage site with the antibody 192swe²² (**Fig 1e**). Furthermore, as compared to wild type mice, increased levels of CTF-η were observed in APPPS-21 brain lysates (**Fig. 1f**). Moreover, the present inventors detected co-staining of antibodies raised against the C-terminus of APP (Y188) with an epitope N-terminal to the Aβ domain (2E9) in dystrophic neurites of 6 month old APPPS-21 brains, but not in areas of aggregated Aβ detected by 6E10 staining in the plaque core (**Fig. 1g****;** similar data were obtained with antibodies 22C11, 9476M, 9478D in human AD brains (**figure 6**)). This suggests that CTF-η may accumulate together with full-length APP and CTF-α/β in dystrophic neurites surrounding neuritic plaques of APPPS-21 mice (**Fig. 1g**). To provide direct evidence for the accumulation of CTF-η in dystrophic neurites, the present inventors used laser capture microdissection (LCM) to differentially enrich for proteins accumulating in dystrophic neurites and plaque cores. Indeed, Western blot analysis revealed not only CTF-β and lower levels of CTF-α, but also CTF-η within the halo of dystrophic neurites and not within the plaque core area or regions devoid of plaques. As expected, Aβ was observed within the plaque core as well as in the surrounding halo (**Fig. 1h**).

Since membrane bound matrix-metalloproteinases like MT1-MMP and MT5-MMP were shown to cleave *in vitro* at a site consistent with η-secretase cleavage^{23,24}, the present inventors produced a neo-epitope specific antibody (10A8; **Fig. 2a**), which allowed identification of the η-cleavage site. Antibody 10A8 identified a fragment corresponding to sAPP-η with an approximate molecular weight of 80 kDa in mouse brain lysates, which is absent in the APP KO brain (**Fig. 2a**). Of note, antibody 10A8 did not identify sAPP-α/β (**Fig. 2a**), demonstrating the selectivity of this antibody for the η-cleavage site. Thus η-secretase cleavage of APP occurs *in vivo* at least in part at amino acids 504/505 (based on APP₆₉₅ numbering; the full APP₆₉₅ sequence is given in SEQ ID NO: 10). Moreover, sAPP-η was found to be significantly increased in supernatants of cells co-expressing APP and MT1-MMP or MT5-MMP (data not shown). To prove which of the two MMPs initiates Aη production *in vivo,* the present inventors investigated brains from MT5- and MT1-MMP -/- mice^{25,26}. Aη generation was reduced in brains from MT5-MMP knockout animals (**Fig. 2b**), whereas a knockout of MT1-MMP had no significant effect on Aη generation (**Fig. 2c**). Thus MT5-MMP displays η-secretase activity in brains, although additional η-secretases cannot be excluded. In fact, the results presented in Fig. 8 demonstrate that also MT1-MMP may act as η-secretase, ultimately resulting in an Aη-α species of 14kDa that is slightly shorter than the Aη-α species that is obtained in cells overexpressing MT5-MMP.

While investigating protease inhibitors capable to modulate η-secretase activity, the present inventors observed that pharmacological BACE1 inhibition led to a pronounced accumulation of the long Aη-α species in CHO cells overexpressing APP_{V717F} (**Fig. 3a**). This indicates that, upon BACE1 inhibition, processing by α-secretase leads to enhanced production of the long Aη-α species to the expense of shorter BACE1 generated Aη-β. Similarly, in primary hippocampal neurons BACE1 inhibition also led to an enhanced production of endogenous Aη-α (**Fig. 3b**). Moreover, analysis of brains from BACE1 -/- mice confirmed a robust increase of endogenous CTF-η and Aη-α *in vivo* (**Fig. 3c**). Finally, pharmacological intervention *in vivo* with a single oral dose of the BACE1 inhibitor RO5508887²⁷ also revealed a significant time dependent increase of CTF-η and Aη-α in APP_{V717I} transgenic mice²⁸ (**Fig. 3d**), which was fully reversible due to clearance of the inhibitor after 24 h.

Since cleavage products of the Aη processing pathway accumulate upon BACE1 inhibition (**Fig. 3**) and are observed in dystrophic neurites (see **Fig. 1g** **and h**), the present inventors thought to investigate whether soluble Aη peptides interfere with neuronal function similar to soluble Aβ oligomers¹. LTP is a correlate of memory²⁹ and is frequently used to investigate neurotoxic effects of Aβ oligomers on learning *ex vivo*^{5,30,31}*.* In order to investigate potential effects of *in vivo* produced Aη peptides on synaptic activity, the present inventors expressed Aη-β and Aη-α in CHO cells and collected conditioned media (**Fig. 4a**) and enriched for these peptides by size exclusion chromatography (SEC). SEC fractions enriched for Aη-β and Aη-α. were applied to hippocampal slices prior to inducing LTP in CA1 pyramidal neurons by high-frequency stimulation. The present inventors compared LTP over 60 min obtained in the presence of Aη-β or Aη-α to LTP obtained under control conditions (**Fig. 4b****-e**). Strikingly, Aη-α inhibited LTP (**Fig. 4c**) to a degree comparable to Aβ_{S26C}³¹ (**figure 7**), whereas truncated Aη-β had no effect (**Fig. 4d****;** quantified in **Fig. 4e**).

### References:

1 Haass, C. & Selkoe, D. J. Soluble protein oligomers in neurodegeneration: lessons from the Alzheimer's amyloid beta-peptide. Nat. Rev. Mol. Cell Biol. 8, 101-112, (2007).
2 Nygaard, H. B. Current and Emerging Therapies for Alzheimer's Disease. Clin. Ther. 35, 1480-1489, (2013).
3 Dobrowolska, J. A. et al. CNS amyloid-beta, soluble APP-alpha and -beta kinetics during BACE inhibition. J. Neurosci. 34, 8336-8346, (2014).
4 Sekine-Aizawa, Y. et al. Matrix metalloproteinase (MMP) system in brain: identification and characterization of brain-specific MMP highly expressed in cerebellum. Eur. J. Neurosci. 13, 935-948, (2001).
5 Walsh, D. M. et al. Naturally secreted oligomers of amyloid beta protein potently inhibit hippocampal long-term potentiation in vivo. Nature 416, 535-539, (2002).
6 Haass, C., Kaether, C., Thinakaran, G. & Sisodia, S. Trafficking and proteolytic processing of APP. Cold Spring Harb Perspect Med 2, a006270, (2012).
7 Willem, M., Lammich, S. & Haass, C. Function, regulation and therapeutic properties of beta-secretase (BACE1). Semin. Cell Dev. Biol. 20, 175-182, (2009).
8 Yan, R. & Vassar, R. Targeting the beta secretase BACE1 for Alzheimer's disease therapy. Lancet Neurol. 13, 319-329, (2014).
9 McConlogue, L. et al. Partial reduction of BACE1 has dramatic effects on Alzheimer plaque and synaptic pathology in APP Transgenic Mice. J. Biol. Chem. 282, 26326-26334, (2007).
10 Postina, R. et al. A disintegrin-metalloproteinase prevents amyloid plaque formation and hippocampal defects in an Alzheimer disease mouse model. J. Clin. Invest. 113, 1456-1464, (2004).
11 Simons, M. et al. Amyloidogenic processing of the human amyloid precursor protein in primary cultures of rat hippocampal neurons. J. Neurosci. 16, 899-908, (1996).
12 Nikolaev, A., McLaughlin, T., O'Leary, D. D. & Tessier-Lavigne, M. APP binds DR6 to trigger axon pruning and neuron death via distinct caspases. Nature 457, 981-989, (2009).
13 Jefferson, T. et al. Metalloprotease meprin beta generates nontoxic N-terminal amyloid precursor protein fragments in vivo. J. Biol. Chem. 286, 27741-27750, (2011).
14 Vella, L. J. & Cappai, R. Identification of a novel amyloid precursor protein processing pathway that generates secreted N-terminal fragments. FASEB J., (2012).
15 Haass, C., Koo, E. H., Mellon, A., Hung, A. Y. & Selkoe, D. J. Targeting of cell-surface beta-amyloid precursor protein to lysosomes: alternative processing into amyloid-bearing fragments. Nature 357, 500-503, (1992).
16 Estus, S. et al. Potentially amyloidogenic, carboxyl-terminal derivatives of the amyloid protein precursor. Science 255, 726-728, (1992).
17 Haass, C. et al. b-Amyloid peptide and a 3-kDa fragment are derived by distinct cellular mechanisms. J. Biol. Chem. 268, 3021-3024, (1993).
18 Muller, U. et al. Behavioral and Anatomical Deficits in Mice Homozygous for a Modified Beta-Amyloid Precursor Protein Gene. Cell 79, 755-765, (1994).
19 Haass, C. et al. Amyloid beta-peptide is produced by cultured cells during normal metabolism. Nature 359, 322-325, (1992).
20 Golde, T. E., Estus, S., Younkin, L. H., Selkoe, D. J. & Younkin, S. G. Processing of the amyloid protein precursor to potentially amyloidogenic derivatives. Science 255, 728-730, (1992).
21 Radde, R. et al. Abeta42-driven cerebral amyloidosis in transgenic mice reveals early and robust pathology. EMBO Rep. 7, 940-946, (2006).
22 Haass, C. et al. The Swedish Mutation Causes Early-Onset Alzheimers-Disease by Beta-Secretase Cleavage within the Secretory Pathway. Nat. Med. 1, 1291-1296, (1995).
23 Higashi, S. & Miyazaki, K. Novel processing of beta-amyloid precursor protein catalyzed by membrane type 1 matrix metalloproteinase releases a fragment lacking the inhibitor domain against gelatinase A. Biochemistry (Mosc.) 42, 6514-6526, (2003).
24 Ahmad, M. et al. Cleavage of amyloid-beta precursor protein (APP) by membrane-type matrix metalloproteinases. J Biochem 139, 517-526, (2006).
25 Folgueras, A. R. et al. Metalloproteinase MT5-MMP is an essential modulator of neuro-immune interactions in thermal pain stimulation. Proc. Natl. Acad. Sci. U. S. A. 106, 16451-16456, (2009).
26 Zhou, Z. et al. Impaired endochondral ossification and angiogenesis in mice deficient in membrane-type matrix metalloproteinase I. Proc. Natl. Acad. Sci. U. S. A. 97, 4052-4057, (2000).
27 Jacobsen, H. et al. Combined treatment with a BACE inhibitor and anti-Ab antibody gantenerumab enhances amyloid reduction in APPLondon mice. J. Neurosci. in press, (2014).
28 Moechars, D. et al. Early phenotypic changes in transgenic mice that overexpress different mutants of amyloid precursor protein in brain. J. Biol. Chem. 274, 6483-6492, (1999).
29 Nabavi, S. et al. Engineering a memory with LTD and LTP. Nature 511, 348-352, (2014).
30 Shankar, G. M. et al. Natural oligomers of the Alzheimer amyloid-beta protein induce reversible synapse loss by modulating an NMDA-type glutamate receptor-dependent signaling pathway. J. Neurosci. 27, 2866-2875, (2007).
31 Shankar, G. M. et al. Amyloid-beta protein dimers isolated directly from Alzheimer's brains impair synaptic plasticity and memory. Nat. Med. 14, 837-842, (2008).
32 Hardy, J. & Selkoe, D. J. The amyloid hypothesis of Alzheimer's disease: progress and problems on the road to therapeutics. Science 297, 353-356, (2002).
33 Haass, C. Take five--BACE and the gamma-secretase quartet conduct Alzheimer's amyloid beta-peptide generation. EMBO J. 23, 483-488, (2004).
34 Jonsson, T. et al. A mutation in APP protects against Alzheimer's disease and age-related cognitive decline. Nature 488, 96-99, (2012).
35 Savonenko, A. V. et al. Alteration of BACE1-dependent NRG1/ErbB4 signaling and schizophrenia-like phenotypes in BACE1-null mice. Proc. Natl. Acad. Sci. U. S. A. 105, 5585-5590, (2008).
36 Kuhn, P. H. et al. Secretome protein enrichment identifies physiological BACE1 protease substrates in neurons. EMBO J. 31, 3157-3168, (2012).
37 Zhou, L. et al. The neural cell adhesion molecules L1 and CHL1 are cleaved by BACE1 protease in vivo. The Journal of biological chemistry 287, 25927-25940, (2012).
38 Blennow, K., Zetterberg, H., Haass, C. & Finucane, T. Semagacestat's fall: where next for AD therapies? Nat. Med. 19, 1214-1215, (2013).
39 Nolan, R. L. & Teller, J. K. Diethylamine extraction of proteins and peptides isolated with a mono-phasic solution of phenol and guanidine isothiocyanate. J. Biochem. Biophys. Methods 68, 127-131, (2006).
40 Westmeyer, G. G. et al. Dimerization of beta-site beta-amyloid precursor protein-cleaving enzyme. J. Biol. Chem. 279, 53205-53212, (2004).
41 Fleck, D. et al. Dual cleavage of neuregulin 1 type III by BACE1 and ADAM17 liberates its EGF-like domain and allows paracrine signaling. J. Neurosci. 33, 7856-7869, (2013).
42 Houeland, G. et al. Transgenic mice with chronic NGF deprivation and Alzheimer's disease-like pathology display hippocampal region-specific impairments in short- and long-term plasticities. J. Neurosci. 30, 13089-13094, (2010).
43 Townsend, M., Shankar, G. M., Mehta, T., Walsh, D. M. & Selkoe, D. J. Effects of secreted oligomers of amyloid beta-protein on hippocampal synaptic plasticity: a potent role for trimers. J Physiol 572, 477-492, (2006).
44 Podlisny, M. B. et al. Aggregation of Secreted Amyloid Beta-Protein into Sodium <Dodecyl Sulfate-Stable Oligomers in Cell-Culture. J. Biol. Chem. 270, 9564-9570, (1995).
45 Kaech, S. & Banker, G. Culturing hippocampal neurons. Nat. Protoc. 1, 2406-2415, (2006).
46 Cai, H. et al. BACE1 is the major beta-secretase for generation of Abeta peptides by neurons. Nat. Neurosci. 4, 233-234, (2001).
47 Radde, R. et al. Abeta42-driven cerebral amyloidosis in transgenic mice reveals early and robust pathology. EMBO Rep. 7, 940-946, (2006).
48 Moechars, D. et al. Early phenotypic changes in transgenic mice that overexpress different mutants of amyloid precursor protein in brain. J. Biol. Chem. 274, 6483-6492, (1999).
49 Jacobsen, H. et al. Combined treatment with a BACE inhibitor and anti-Ab antibody gantenerumab enhances amyloid reduction in APPLondon mice. J. Neurosci., (2014); 34(35): 11621-11630.
50 Nolan, R. L. & Teller, J. K. Diethylamine extraction of proteins and peptides isolated with a mono-phasic solution of phenol and guanidine isothiocyanate. J. Biochem. Biophys. Methods 68, 127-131, (2006).
51 Westmeyer, G. G. et al. Dimerization of beta-site beta-amyloid precursor protein-cleaving enzyme. J. Biol. Chem. 279, 53205-53212, (2004).
52 Braak, H. & Braak, E. Neuropathological stageing of Alzheimer-related changes. Acta Neuropathol 82, 239-259, (1991).
53 Mirra, S. S. et al. The Consortium to Establish a Registry for Alzheimer's Disease (CERAD). Part II. Standardization of the neuropathologic assessment of Alzheimer's disease. Neurology 41, 479-486, (1991).
54 Hyman, B. T. et al. National Institute on Aging-Alzheimer's Association guidelines for the neuropathologic assessment of Alzheimer's disease. Alzheimers Dement 8, 1-13, (2012).
55 Liao, L. et al. Proteomic characterization of postmortem amyloid plaques isolated by laser capture microdissection. J. Biol. Chem. 279, 37061-37068, (2004).
56 Houeland, G. et al. Transgenic mice with chronic NGF deprivation and Alzheimer's disease-like pathology display hippocampal region-specific impairments in short- and long-term plasticities. J. Neurosci. 30, 13089-13094, (2010).
57 Townsend, M., Shankar, G. M., Mehta, T., Walsh, D. M. & Selkoe, D. J. Effects of secreted oligomers of amyloid beta-protein on hippocampal synaptic plasticity: a potent role for trimers. J Physiol 572, 477-492, (2006).

The features of the present invention disclosed in the specification, the claims, and/or in the accompanying drawings may, both separately and in any combination thereof, be material for realizing the invention in various forms thereof.

## Claims

1. A peptide or collection of peptides that is the reaction product of matrix-metalloproteinase MT1-MMP or MT5-MMPacting on amyloid precursor protein (APP), followed by the action of both α-secretase (ADAM10) and β-secretase (β-site APP cleaving enzyme 1; BACE1).

2. The peptide or collection of peptides according to claim 1, having one or several epitopes selected from MISEPRISYG (SEQ ID NO: 1), DALMPSLT (SEQ ID NO: 2), PWHSFGADSVP (SEQ ID NO: 3), SEVKM (SEQ ID NO: 4), and DAEFRHDSGYEVHHQK (SEQ ID NO: 5).

3. The peptide or collection of peptides according to any of claims 1-2, **characterized by** recognition through an antibody which recognizes one or several of said epitopes of claim 2.

4. The peptide or collection of peptides according to any of claims 1-3, having a sequence selected from MISEPRISYGNDALMPSLTETKTTVELLPVNGEFSLDDLQPWHSFGADSVPANT ENEVEPVDARPAADRGLTTRPGSGLTNIKTEEISEVKM (SEQ ID NO: 6) and MISEPRISYGNDALMPSLTETKTTVELLPVNGEFSLDDLQPWHSFGADSVPANT ENEVEPVDARPAADRGLTTRPGSGLTNIKTEEISEVKMDAEFRHDSGYEVHHQ K (SEQ ID NO: 7).

5. The peptide or collection of peptides according to claim 4, wherein one peptide has a sequence MISEPRISYGNDALMPSLTETKTTVELLPVNGEFSLDDLQPWHSFGADSVPANT ENEVEPVDARPAADRGLTTRPGSGLTNIKTEEISEVKM (SEQ ID NO: 6) and another peptide has a sequence MISEPRISYGNDALMPSLTETKTTVELLPVNGEFSLDDLQPWHSFGADSVPANT ENEVEPVDARPAADRGLTTRPGSGLTNIKTEEISEVKMDAEFRHDSGYEVHHQ K (SEQ ID NO: 7).

6. A composition of peptides comprising one or several peptides according to any of claims 1-5 and at least one further peptide that is the reaction product of matrix-metalloproteinase MT1-MMP or MT5-MMP only on amyloid precursor protein (APP).

7. The composition of peptides according to claim 6, wherein the at least one further peptide is membrane bound or is soluble in aqueous solution.

8. The composition of peptides according to any of claims 6-7, comprising both a membrane bound further peptide that is the reaction product of matrix-metalloproteinase MT1-MMP or MT5-MMP only acting on amyloid precursor protein (APP), and a peptide soluble in aqueous solution and being the reaction product of matrix-metalloproteinase MT1-MMP or MT5-MMP only acting on amyloid precursor protein (APP).

9. The composition of peptides according to claim 8, wherein said membrane bound further peptide has a sequence represented by SEQ ID NO: 8, and said peptide soluble in aqueous solution has a sequence represented by SEQ ID NO. 9.

10. The peptide or collection of peptides according to any of claims 1-5 or the composition of peptides according to any claims 6-9, for use in a method of detecting Alzheimer's disease in a patient, wherein said method involves detecting said peptides or said collection of peptides.

11. The peptide or collection or composition of peptides for use according to claim 10, wherein said use involves the detection of said peptide or of said collection or composition of peptides in cerebrospinal fluid (CSF) or plasma.

12. The peptide or collection of peptides according to any of claims 1-5 or the composition of peptides according to any claims 6-9, for use in a method of treatment, prevention and/or alleviation of Alzheimer's disease, wherein said method involves the inhibition, in a patient, of formation or action of said peptide or said collection of peptides or of said composition of peptides, or the inhibition of action or production of matrix-metalloproteinase MT5-MMP and/or MT1-MMP.

## Patentansprüche

1. Peptid oder Peptidsammlung, das/die das Reaktionsprodukt von Matrix-Metalloproteinase MT1-MMP oder MT5-MMP ist, die auf das Amyloid-Vorläuferprotein (APP) wirkt, gefolgt von der Wirkung von sowohl α-Sekretase (ADAM10) als auch β-Sekretase (β-Stelle APP spaltendes Enzym; BACE1).

2. Peptid oder Peptidsammlung nach Anspruch 1, das/die ein oder mehrere Epitop(e) hat, ausgewählt aus MISEPRISYG (SEQ ID Nr: 1), DALMPSLT (SEQ ID Nr: 2), PWHSFGADSVP (SEQ ID Nr: 3), SEVKM (SEQ ID Nr: 4) und DAEFRHDSGYEVHHQK (SEQ ID Nr: 5).

3. Peptid oder Peptidsammlung nach einem der Ansprüche 1-2, **gekennzeichnet durch** die Erkennung durch einen Antikörper, der eines oder mehrere der Epitope nach Anspruch 2 erkennt.

4. Peptid oder Peptidsammlung nach einem der Ansprüche 1-3, das/die eine Sequenz hat, ausgewählt aus MISEPRISYGNDALMPSLTETKTTVELLPVNGEFSLDDLQPWHSFGADSVPANT ENEVEPVDARPAADRGLTTRPGSGLTNIKTEEISEVKM (SEQ ID Nr: 6) und MISEPRISYGNDALMPSLTETKTTVELLPVNGEFSLDDLQPWHSFGADSVPANT ENEVEPVDARPAADRGLTTRPGSGLTNIKTEEISEVKMDAEFRHDSGYEVHHQ K (SEQ ID Nr: 7).

5. Peptid oder Peptidsammlung nach Anspruch 4, wobei ein Peptid eine Sequenz MISEPRISYGNDALMPSLTETKTTVELLPVNGEFSLDDLQPWHSFGADSVPANT ENEVEPVDARPAADRGLTTRPGSGLTNIKTEEISEVKM (SEQ ID Nr: 6) hat und ein weiteres Peptid eine Sequenz MISEPRISYGNDALMPSLTETKTTVELLPVNGEFSLDDLQPWHSFGADSVPANT ENEVEPVDARPAADRGLTTRPGSGLTNIKTEEISEVKMDAEFRHDSGYEVHHQ K (SEQ ID Nr: 7) hat.

6. Peptidzusammensetzung, umfassend ein oder mehrere Peptid(e) nach einem der Ansprüche 1-5 und mindestens ein weiteres Peptid, das das Reaktionsprodukt von ausschließlich Matrix-Metalloproteinase MT1-MMP oder MT5-MMP an dem Amyloid-Vorläuferprotein (APP) ist.

7. Peptidzusammensetzung nach Anspruch 6, wobei das mindestens eine weitere Peptid membrangebunden ist oder in wässriger Lösung löslich ist.

8. Peptidzusammensetzung nach einem der Ansprüche 6-7, umfassend sowohl ein membrangebundenes weiteres Peptid, das das Reaktionsprodukt von ausschließlich Matrix-Metalloproteinase MT1-MMP oder MT5-MMP ist, die auf das Amyloid-Vorläuferprotein (APP) wirkt, als auch ein Peptid, das in wässriger Lösung löslich ist und das das Reaktionsprodukt von ausschließlich Matrix-Metalloproteinase MT1-MMP oder MT5-MMP ist, die auf das Amyloid-Vorläuferprotein (APP) wirkt.

9. Peptidzusammensetzung nach Anspruch 8, wobei das membrangebundene weitere Peptid eine durch SEQ ID Nr: 8 dargestellte Sequenz hat und das in wässriger Lösung lösliche Peptid eine durch SEQ ID Nr: 9 dargestellte Sequenz hat.

10. Peptid oder Peptidsammlung nach einem der Ansprüche 1-5 oder die Peptidzusammensetzung nach einem der Ansprüche 6-9 zur Verwendung in einem Verfahren zum Nachweisen der Alzheimer-Krankheit bei einem Patienten, wobei das Verfahren das Nachweisen der Peptide oder der Peptidsammlung umfasst.

11. Peptid, oder Peptidsammlung oder -zusammensetzung, zur Verwendung nach Anspruch 10, wobei die Verwendung das Nachweisen des Peptids, oder der Peptidsammlung oder -zusammensetzung, in Cerebrospinalflüssigkeit (CSF) oder Plasma umfasst.

12. Peptid oder Peptidsammlung nach einem der Ansprüche 1-5 oder Peptidzusammensetzung nach einem der Ansprüche 6-9 zur Verwendung in einem Verfahren zum Behandeln, Verhindern und/oder Lindern der Alzheimer-Krankheit, wobei das Verfahren umfasst: die Inhibition, bei einem Patienten, der Bildung oder Wirkung des Peptids oder der Peptidsammlung oder der Peptidzusammensetzung, oder die Inhibition der Wirkung oder Produktion von Matrix-Metalloproteinase MT5-MMP und/oder MT1-MMP.

## Revendications

1. Peptide ou collection de peptides qui est le produit réactionnel de la métalloprotéase matricielle MT1-MMP ou MT5-MMP agissant sur la protéine précurseur de l'amyloïde (APP), suivi de l'action à la fois de l'α-sécrétase (ADAM10) et de la β-sécrétase (enzyme 1 de clivage de l'APP au niveau du site β ; BACE1).

2. Peptide ou collection de peptides selon la revendication 1, ayant un ou plusieurs épitopes choisis parmi MISEPRISYG (SEQ ID N° : 1), DALMPSLT (SEQ ID N° : 2), PWHSFGADSVP (SEQ ID N° : 3), SEVKM (SEQ ID N° : 4) et DAEFRHDSGYEVHHQK (SEQ ID NO : 5).

3. Peptide ou collection de peptides selon l'une quelconque des revendications 1 à 2, **caractérisé par** une reconnaissance par le biais d'un anticorps qui reconnaît un ou plusieurs desdits épitopes selon la revendication 2.

4. Peptide ou collection de peptides selon l'une quelconque des revendications 1 à 3, ayant une séquence choisie parmi MISEPRISYGNDALMPSLTETKTTVELLPVNGEFSLDDLQPWHSFGADSVPANTE NEVEPVDARPAADRGLTTRPGSGLTNIKTEEISEVKM (SEQ ID N° : 6) et MISEPRISYGNDALMPSLTETKTTVELLPVNGEFSLDDLQPWHSFGADSVPANTE NEVEPVDARPAADRGLTTRPGSGLTNIKTEEISEVKMDAEFRHDSGYEVHHQK ( SEQ ID N° : 7).

5. Peptide ou collection de peptides selon la revendication 4, dans lequel un peptide a une séquence MISEPRISYGNDALMPSLTETKTTVELLPVNGEFSLDDLQPWHSFGADSVPANTE NEVEPVDARPAADRGLTTRPGSGLTNIKTEEISEVKM (SEQ ID N° : 6) et un autre peptide a une séquence MISEPRISYGNDALMPSLTETKTTVELLPVNGEFSLDDLQPWHSFGADSVPANTE NEVEPVDARPAADRGLTTRPGSGLTNIKTEEISEVKMDAEFRHDSGYEVHHQK ( SEQ ID N° : 7).

6. Composition de peptides comprenant un ou plusieurs peptides selon l'une quelconque des revendications 1 à 5 et au moins un peptide supplémentaire qui est le produit réactionnel de la métalloprotéase matricielle MT1-MMP ou MT5-MMP uniquement sur la protéine précurseur de l'amyloïde (APP).

7. Composition de peptides selon la revendication 6, dans laquelle l'au moins un peptide supplémentaire est lié à la membrane ou est soluble en solution aqueuse.

8. Composition de peptides selon l'une quelconque des revendications 6 à 7, comprenant à la fois un peptide supplémentaire lié à la membrane qui est le produit réactionnel de la métalloprotéase matricielle MT1-MMP ou MT5-MMP agissant uniquement sur la protéine précurseur de l'amyloïde (APP), et un peptide soluble en solution aqueuse et étant le produit réactionnel de la métalloprotéase matricielle MT1-MMP ou MT5-MMP agissant uniquement sur la protéine précurseur de l'amyloïde (APP).

9. Composition de peptides selon la revendication 8, dans laquelle ledit peptide supplémentaire lié à la membrane a une séquence représentée par la SEQ ID N° : 8, et ledit peptide soluble en solution aqueuse a une séquence représentée par la SEQ ID N° : 9.

10. Peptide ou collection de peptides selon l'une quelconque des revendications 1 à 5 ou composition de peptides selon l'une quelconque des revendications 6 à 9, pour une utilisation dans un procédé de détection de la maladie d'Alzheimer chez un patient, dans lequel ledit procédé implique la détection desdits peptides ou de ladite collection de peptides.

11. Peptide ou collection ou composition de peptides pour une utilisation selon la revendication 10, dans lequel ladite utilisation implique la détection dudit peptide ou de ladite collection ou composition de peptides dans le liquide céphalorachidien (CSF) ou le plasma.

12. Peptide ou collection de peptides selon l'une quelconque des revendications 1 à 5 ou composition de peptides selon l'une quelconque des revendications 6 à 9, pour une utilisation dans un procédé de traitement, de prévention et/ou de soulagement de la maladie d'Alzheimer, dans lequel ledit procédé implique l'inhibition, chez un patient, de la formation ou de l'action dudit peptide ou de ladite collection de peptides ou de ladite composition de peptides, ou l'inhibition de l'action ou de la production de la métalloprotéase matricielle MT5-MMP et/ou MT1-MMP.


### SEQUENCE LISTING

<110> Ludwig-Maximilians-Universitat München
<120> A peptide or collection of peptides derived from amyloid precursor protein
<130> U30612WO
<160> 10
<170> PatentIn version 3.5
<210> 1
   <211> 10
   <212> PRT
   <213> Homo sapiens
<400> 1
   Met Ile Ser Glu Pro Arg Ile Ser Tyr Gly
   1               5                   10
<210> 2
   <211> 8
   <212> PRT
   <213> Homo sapiens
<400> 2
   Asp Ala Leu Met Pro Ser Leu Thr
   1               5
<210> 3
   <211> 11
   <212> PRT
   <213> Homo sapiens
<400> 3
   Pro Trp His Ser Phe Gly Ala Asp Ser Val Pro
   1               5                   10
<210> 4
   <211> 5
   <212> PRT
   <213> Homo sapiens
<400> 4
   Ser Glu Val Lys Met
   1               5
<210> 5
   <211> 16
   <212> PRT
   <213> Homo sapiens
<400> 5
   Asp Ala Glu Phe Arg His Asp Ser Gly Tyr Glu Val His His Gln Lys
   1               5                   10                  15
<210> 6
   <211> 92
   <212> PRT
   <213> Homo sapiens
<400> 6
   Met Ile Ser Glu Pro Arg Ile Ser Tyr Gly Asn Asp Ala Leu Met Pro
   1               5                   10                  15
   Ser Leu Thr Glu Thr Lys Thr Thr Val Glu Leu Leu Pro Val Asn Gly
               20                  25                  30
   Glu Phe Ser Leu Asp Asp Leu Gln Pro Trp His Ser Phe Gly Ala Asp
           35                  40                  45
   Ser Val Pro Ala Asn Thr Glu Asn Glu Val Glu Pro Val Asp Ala Arg
       50                  55                  60
   Pro Ala Ala Asp Arg Gly Leu Thr Thr Arg Pro Gly Ser Gly Leu Thr
   65                  70                  75                  80
   Asn Ile Lys Thr Glu Glu Ile Ser Glu Val Lys Met
                   85                  90
<210> 7
   <211> 108
   <212> PRT
   <213> Homo sapiens
<400> 7
   Met Ile Ser Glu Pro Arg Ile Ser Tyr Gly Asn Asp Ala Leu Met Pro
   1               5                   10                  15
   Ser Leu Thr Glu Thr Lys Thr Thr Val Glu Leu Leu Pro Val Asn Gly
               20                  25                  30
   Glu Phe Ser Leu Asp Asp Leu Gln Pro Trp His Ser Phe Gly Ala Asp
           35                  40                  45
   Ser Val Pro Ala Asn Thr Glu Asn Glu Val Glu Pro Val Asp Ala Arg
       50                  55                  60
   Pro Ala Ala Asp Arg Gly Leu Thr Thr Arg Pro Gly Ser Gly Leu Thr
   65                  70                  75                  80
   Asn Ile Lys Thr Glu Glu Ile Ser Glu Val Lys Met Asp Ala Glu Phe
                   85                  90                  95
   Arg His Asp Ser Gly Tyr Glu Val His His Gln Lys
               100                 105
<210> 8
   <211> 191
   <212> PRT
   <213> Homo sapiens
<400> 8
   Met Ile Ser Glu Pro Arg Ile Ser Tyr Gly Asn Asp Ala Leu Met Pro
   1               5                   10                  15
   Ser Leu Thr Glu Thr Lys Thr Thr Val Glu Leu Leu Pro Val Asn Gly
               20                  25                  30
   Glu Phe Ser Leu Asp Asp Leu Gln Pro Trp His Ser Phe Gly Ala Asp
           35                  40                  45
   Ser Val Pro Ala Asn Thr Glu Asn Glu Val Glu Pro Val Asp Ala Arg
       50                  55                  60
   Pro Ala Ala Asp Arg Gly Leu Thr Thr Arg Pro Gly Ser Gly Leu Thr
   65                  70                  75                  80
   Asn Ile Lys Thr Glu Glu Ile Ser Glu Val Lys Met Asp Ala Glu Phe
                   85                  90                  95
   Arg His Asp Ser Gly Tyr Glu Val His His Gln Lys Leu Val Phe Phe
               100                 105                 110
   Ala Glu Asp Val Gly Ser Asn Lys Gly Ala Ile Ile Gly Leu Met Val
           115                 120                 125
   Gly Gly Val Val Ile Ala Thr Val Ile Val Ile Thr Leu Val Met Leu
       130                 135                 140
   Lys Lys Lys Gln Tyr Thr Ser Ile His His Gly Val Val Glu Val Asp
   145                 150                 155                 160
   Ala Ala Val Thr Pro Glu Glu Arg His Leu Ser Lys Met Gln Gln Asn
                   165                 170                 175
   Gly Tyr Glu Asn Pro Thr Tyr Lys Phe Phe Glu Gln Met Gln Asn
               180                 185                 190
<210> 9
   <211> 486
   <212> PRT
   <213> Homo sapiens
<400> 9
   Glu Val Pro Thr Asp Gly Asn Ala Gly Leu Leu Ala Glu Pro Gln Ile
   1               5                   10                  15
   Ala Met Phe Cys Gly Arg Leu Asn Met His Met Asn Val Gln Asn Gly
               20                  25                  30
   Lys Trp Asp Ser Asp Pro Ser Gly Thr Lys Thr Cys Ile Asp Thr Lys
           35                  40                  45
   Glu Gly Ile Leu Gln Tyr Cys Gln Glu Val Tyr Pro Glu Leu Gln Ile
       50                  55                  60
   Thr Asn Val Val Glu Ala Asn Gln Pro Val Thr Ile Gln Asn Trp Cys
   65                  70                  75                  80
   Lys Arg Gly Arg Lys Gln Cys Lys Thr His Pro His Phe Val Ile Pro
                   85                  90                  95
   Tyr Arg Cys Leu Val Gly Glu Phe Val Ser Asp Ala Leu Leu Val Pro
               100                 105                 110
   Asp Lys Cys Lys Phe Leu His Gln Glu Arg Met Asp Val Cys Glu Thr
           115                 120                 125
   His Leu His Trp His Thr Val Ala Lys Glu Thr Cys Ser Glu Lys Ser
       130                 135                 140
   Thr Asn Leu His Asp Tyr Gly Met Leu Leu Pro Cys Gly Ile Asp Lys
   145                 150                 155                 160
   Phe Arg Gly Val Glu Phe Val Cys Cys Pro Leu Ala Glu Glu Ser Asp
                   165                 170                 175
   Asn Val Asp Ser Ala Asp Ala Glu Glu Asp Asp Ser Asp Val Trp Trp
               180                 185                 190
   Gly Gly Ala Asp Thr Asp Tyr Ala Asp Gly Ser Glu Asp Lys Val Val
           195                 200                 205
   Glu Val Ala Glu Glu Glu Glu Val Ala Glu Val Glu Glu Glu Glu Ala
       210                 215                 220
   Asp Asp Asp Glu Asp Asp Glu Asp Gly Asp Glu Val Glu Glu Glu Ala
   225                 230                 235                 240
   Glu Glu Pro Tyr Glu Glu Ala Thr Glu Arg Thr Thr Ser Ile Ala Thr
                   245                 250                 255
   Thr Thr Thr Thr Thr Thr Glu Ser Val Glu Glu Val Val Arg Val Pro
               260                 265                 270
   Thr Thr Ala Ala Ser Thr Pro Asp Ala Val Asp Lys Tyr Leu Glu Thr
           275                 280                 285
   Pro Gly Asp Glu Asn Glu His Ala His Phe Gln Lys Ala Lys Glu Arg
       290                 295                 300
   Leu Glu Ala Lys His Arg Glu Arg Met Ser Gln Val Met Arg Glu Trp
   305                 310                 315                 320
   Glu Glu Ala Glu Arg Gln Ala Lys Asn Leu Pro Lys Ala Asp Lys Lys
                   325                 330                 335
   Ala Val Ile Gln His Phe Gln Glu Lys Val Glu Ser Leu Glu Gln Glu
               340                 345                 350
   Ala Ala Asn Glu Arg Gln Gln Leu Val Glu Thr His Met Ala Arg Val
           355                 360                 365
   Glu Ala Met Leu Asn Asp Arg Arg Arg Leu Ala Leu Glu Asn Tyr Ile
       370                 375                 380
   Thr Ala Leu Gln Ala Val Pro Pro Arg Pro Arg His Val Phe Asn Met
   385                 390                 395                 400
   Leu Lys Lys Tyr Val Arg Ala Glu Gln Lys Asp Arg Gln His Thr Leu
                   405                 410                 415
   Lys His Phe Glu His Val Arg Met Val Asp Pro Lys Lys Ala Ala Gln
               420                 425                 430
   Ile Arg Ser Gln Val Met Thr His Leu Arg Val Ile Tyr Glu Arg Met
           435                 440                 445
   Asn Gln Ser Leu Ser Leu Leu Tyr Asn Val Pro Ala Val Ala Glu Glu
       450                 455                 460
   Ile Gln Asp Glu Val Asp Glu Leu Leu Gln Lys Glu Gln Asn Tyr Ser
   465                 470                 475                 480
   Asp Asp Val Leu Ala Asn
   485
<210> 10
   <211> 695
   <212> PRT
   <213> Homo sapiens
<400> 10
   Met Leu Pro Gly Leu Ala Leu Leu Leu Leu Ala Ala Trp Thr Ala Arg
   1               5                   10                  15
   Ala Leu Glu Val Pro Thr Asp Gly Asn Ala Gly Leu Leu Ala Glu Pro
               20                  25                  30
   Gln Ile Ala Met Phe Cys Gly Arg Leu Asn Met His Met Asn Val Gln
           35                  40                  45
   Asn Gly Lys Trp Asp Ser Asp Pro Ser Gly Thr Lys Thr Cys Ile Asp
       50                  55                  60
   Thr Lys Glu Gly Ile Leu Gln Tyr Cys Gln Glu Val Tyr Pro Glu Leu
   65                  70                  75                  80
   Gln Ile Thr Asn Val Val Glu Ala Asn Gln Pro Val Thr Ile Gln Asn
                   85                  90                  95
   Trp Cys Lys Arg Gly Arg Lys Gln Cys Lys Thr His Pro His Phe Val
               100                 105                 110
   Ile Pro Tyr Arg Cys Leu Val Gly Glu Phe Val Ser Asp Ala Leu Leu
           115                 120                 125
   Val Pro Asp Lys Cys Lys Phe Leu His Gln Glu Arg Met Asp Val Cys
       130                 135                 140
   Glu Thr His Leu His Trp His Thr Val Ala Lys Glu Thr Cys Ser Glu
   145                 150                 155                 160
   Lys Ser Thr Asn Leu His Asp Tyr Gly Met Leu Leu Pro Cys Gly Ile
                   165                 170                 175
   Asp Lys Phe Arg Gly Val Glu Phe Val Cys Cys Pro Leu Ala Glu Glu
               180                 185                 190
   Ser Asp Asn Val Asp Ser Ala Asp Ala Glu Glu Asp Asp Ser Asp Val
           195                 200                 205
   Trp Trp Gly Gly Ala Asp Thr Asp Tyr Ala Asp Gly Ser Glu Asp Lys
       210                 215                 220
   Val Val Glu Val Ala Glu Glu Glu Glu Val Ala Glu Val Glu Glu Glu
   225                 230                 235                 240
   Glu Ala Asp Asp Asp Glu Asp Asp Glu Asp Gly Asp Glu Val Glu Glu
                   245                 250                 255
   Glu Ala Glu Glu Pro Tyr Glu Glu Ala Thr Glu Arg Thr Thr Ser Ile
               260                 265                 270
   Ala Thr Thr Thr Thr Thr Thr Thr Glu Ser Val Glu Glu Val Val Arg
           275                 280                 285
   Val Pro Thr Thr Ala Ala Ser Thr Pro Asp Ala Val Asp Lys Tyr Leu
       290                 295                 300
   Glu Thr Pro Gly Asp Glu Asn Glu His Ala His Phe Gln Lys Ala Lys
   305                 310                 315                 320
   Glu Arg Leu Glu Ala Lys His Arg Glu Arg Met Ser Gln Val Met Arg
                   325                 330                 335
   Glu Trp Glu Glu Ala Glu Arg Gln Ala Lys Asn Leu Pro Lys Ala Asp
               340                 345                 350
   Lys Lys Ala Val Ile Gln His Phe Gln Glu Lys Val Glu Ser Leu Glu
           355                 360                 365
   Gln Glu Ala Ala Asn Glu Arg Gln Gln Leu Val Glu Thr His Met Ala
       370                 375                 380
   Arg Val Glu Ala Met Leu Asn Asp Arg Arg Arg Leu Ala Leu Glu Asn
   385                 390                 395                 400
   Tyr Ile Thr Ala Leu Gln Ala Val Pro Pro Arg Pro Arg His Val Phe
                   405                 410                 415
   Asn Met Leu Lys Lys Tyr Val Arg Ala Glu Gln Lys Asp Arg Gln His
               420                 425                 430
   Thr Leu Lys His Phe Glu His Val Arg Met Val Asp Pro Lys Lys Ala
           435                 440                 445
   Ala Gln Ile Arg Ser Gln Val Met Thr His Leu Arg Val Ile Tyr Glu
       450                 455                 460
   Arg Met Asn Gln Ser Leu Ser Leu Leu Tyr Asn Val Pro Ala Val Ala
   465                 470                 475                 480
   Glu Glu Ile Gln Asp Glu Val Asp Glu Leu Leu Gln Lys Glu Gln Asn
                   485                 490                 495
   Tyr Ser Asp Asp Val Leu Ala Asn Met Ile Ser Glu Pro Arg Ile Ser
               500                 505                 510
   Tyr Gly Asn Asp Ala Leu Met Pro Ser Leu Thr Glu Thr Lys Thr Thr
           515                 520                 525
   Val Glu Leu Leu Pro Val Asn Gly Glu Phe Ser Leu Asp Asp Leu Gln
       530                 535                 540
   Pro Trp His Ser Phe Gly Ala Asp Ser Val Pro Ala Asn Thr Glu Asn
   545                 550                 555                 560
   Glu Val Glu Pro Val Asp Ala Arg Pro Ala Ala Asp Arg Gly Leu Thr
                   565                 570                 575
   Thr Arg Pro Gly Ser Gly Leu Thr Asn Ile Lys Thr Glu Glu Ile Ser
               580                 585                 590
   Glu Val Lys Met Asp Ala Glu Phe Arg His Asp Ser Gly Tyr Glu Val
           595                 600                 605
   His His Gln Lys Leu Val Phe Phe Ala Glu Asp Val Gly Ser Asn Lys
       610                 615                 620
   Gly Ala Ile Ile Gly Leu Met Val Gly Gly Val Val Ile Ala Thr Val
   625                 630                 635                 640
   Ile Val Ile Thr Leu Val Met Leu Lys Lys Lys Gln Tyr Thr Ser Ile
                   645                 650                 655
   His His Gly Val Val Glu Val Asp Ala Ala Val Thr Pro Glu Glu Arg
               660                 665                 670
   His Leu Ser Lys Met Gln Gln Asn Gly Tyr Glu Asn Pro Thr Tyr Lys
           675                 680                 685
   Phe Phe Glu Gln Met Gln Asn
       690                 695